(19) Europäisches Patentamt
European Patent Office
Office européen des brevets

(11) Veröffentlichungsnummer: **0 209 707**
**A2**

(12) # EUROPÄISCHE PATENTANMELDUNG

(21) Anmeldenummer: 86107969.7

(22) Anmeldetag: 11.06.86

(51) Int. Cl.[4]: C 07 D 235/18
C 07 D 471/04, C 07 D 473/32
C 07 D 473/08, C 07 D 473/00
C 07 D 235/02, A 61 K 31/415
A 61 K 31/52, A 61 K 31/495

(30) Priorität: 21.06.85 DE 3522230

(43) Veröffentlichungstag der Anmeldung:
28.01.87 Patentblatt 87/5

(84) Benannte Vertragsstaaten:
AT BE CH DE FR GB IT LI LU NL SE

(71) Anmelder: Dr. Karl Thomae GmbH
Postfach 1755
D-7950 Biberach (Riss)(DE)

(72) Erfinder: Müller, Erich, Dr. Dipl.-Chem.
Talfeldstrasse 34
D-7950 Biberach 1(DE)

(72) Erfinder: Hauel, Norbert, Dr. Dipl.-Chem.
Händelstrasse 12
D-7950 Biberach 1(DE)

(72) Erfinder: Noll, Klaus, Dr. Dipl.-Chem.
Im Schönblick 3
D-7951 Warthausen 1(DE)

(72) Erfinder: Narr, Berthold, Dr. Dipl.-Chem.
Obere Au 5
D-7950 Biberach 1(DE)

(72) Erfinder: Heider, Joachim, Dr. Dipl.-Chem.
Am Hang 3
D-7951 Warthausen(DE)

(72) Erfinder: Psiorz, Manfred, Dr. Dipl.-Chem.
Riedlinger Strasse 35
D-7950 Biberach 1(DE)

(72) Erfinder: Bomhard, Andreas, Dr. Dipl.-Chem.
Dinglingerstrasse 9
D-7950 Biberach 1(DE)

(72) Erfinder: van Meel, Jacques, Dr.
Amriswilstrasse 7
D-7950 Biberach 1(DE)

(72) Erfinder: Diederen, Willi, Dr.
Haldenstrasse 1a
D-7950 Biberach 1(DE)

(54) **Neue 2-Arylimidazole, diese Verbindungen enthaltende Arzneimittel und Verfahren zu ihrer Herstellung.**

(57) Die Erfindung betrifft neue 2-Arylimidazole der allgemeinen Formel

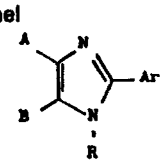

(I)

in der
R ein Wasserstoffatom oder eine Alkylgruppe,
Ar eine Gruppe der Formel

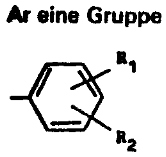

oder

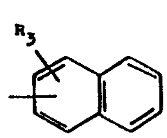

und

A und B zusammen mit den beiden dazwischen liegenden Kohlenstoffatomen eine Gruppe der Formel

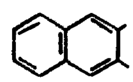
,
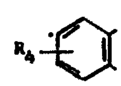
,
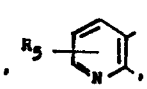
,
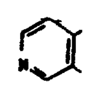
,

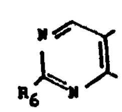

oder

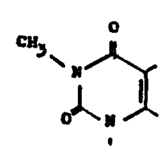

darstellen,

wobei
$R_1$ eine Alkylsulfenyl-, Alkylsulfinyl-, Alkylsulfonyl- oder Alkylsulfoximinogruppe, eine am Stickstoffatom durch eine Alkanoyl-, Alkylsulfonyl- oder Hydroxycarbonylalkylencarbonylgruppe substituierte Alkylsulfoximinogruppe, eine Ethoxy- oder n-Propoxygruppe, die jeweils endständig durch eine Alkylsulfenyl-, Alkylsulfinyl-, Alkylsulfonyl- oder Alkylsulfoximinogruppe substituiert ist, eine Alkoxycar- ./..

EP 0 209 707 A2

Croydon Printing Company Ltd.

bonylamino- oder N-Alkylaminocarbonyl-aminogruppe oder auch in 4-Stellung eine Hydroxy-, Phenylalkoxy-, Aminosulfonyl-, Alkylsulfonyloxy- oder Alkylsulfonylaminogruppe,

$R_2$ ein Wasserstoffatom oder eine Alkoxygruppe,

$R_3$ eine Ethoxy- oder n-Propoxygruppe, die jeweils endständig durch eine Alkylsulfenyl-, Alkylsulfinyl-, Alkylsulfonyl- oder Alkylsulfoximinogruppe substituiert ist,

$R_4$ eine Alkyl-, Alkoxycarbonyl-, Alkanoyl-, N-Alkanoylaminomethyl-, Benzoyl-, Phenyl-methoxymethyl-, Aminocarbonyl-, Cyano, Trifluormethyl-, Aminosulfonyl-, N-Alkylaminocarbonyl-amino- oder N-Alkyl-Nalkyl-aminocarbonyl-aminogruppe.

$R_5$ ein Wasserstoff- oder Halogenatom, eine Alkyl- oder Cyanogruppe und

$R_6$ ein Wasserstoffatom oder eine Aminogruppe bedeuten, sowie die Verbindungen

5-Cyano-2-(4'-methansulfonylamino-2'-methoxy-phenyl)-benzimidazol,

4-Methyl-2-(4'-methansulfonyloxy-2'-methoxy-phenyl)-benzimidazol,

4-Cyano-2-(4'-methansulfonyloxy-2'-methoxy-phenyl)-benzimidazol,

4-Aminocarbonyl-2-(4'-methansulfonyloxy-2'-methoxy-phenyl)-benzimidazol und

4-Methoxycarbonyl-2-(4'-methansulfonyloxy-2'-methoxy-phenyl)-benzimidazol,

und deren Tautomere, wenn R ein Wasserstoffatom darstellt, und deren Säureadditionssalze.

Die neuen Verbindungen und deren physiologisch verträgliche Säureadditionssalze weisen wertvolle pharmakologische Eigenschaften auf, insbesondere eine Wirkung auf den Blutdruck und auf die Kontraktilität des Herzmuskels sowie antithrombotische Wirkungen und lassen sich nach an und für sich bekannten Verfahren herstellen.

DR. KARL THOMAE GMBH
D-7950 Biberach 1



Case 5/928

# Neue 2-Arylimidazole, diese Verbindungen enthaltende Arzneimittel und Verfahren zu ihrer Herstellung

Gegenstand der vorliegenden Erfindung sind neue 2-Arylimidazole der allgemeinen Formel

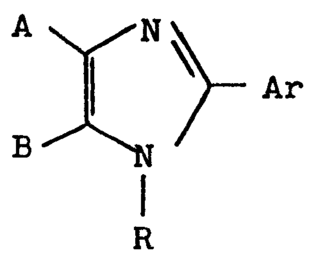

(I)

und deren Tautomere, wenn R ein Wasserstoffatom darstellt, sowie die Verbindungen

5-Cyano-2-(4'-methansulfonylamino-2'-methoxy-phenyl)-benzimidazol,

4-Methyl-2-(4'-methansulfonyloxy-2'-methoxy-phenyl)-benzimidazol,

4-Cyano-2-(4'-methansulfonyloxy-2'-methoxy-phenyl)-benzimidazol,

4-Aminocarbonyl-2-(4'-methansulfonyloxy-2'-methoxy-phenyl)-benzimidazol und

4-Methoxycarbonyl-2-(4'-methansulfonyloxy-2'-methoxy-phenyl)-benzimidazol

und deren Säureadditionssalze, insbesondere deren physiologisch verträgliche Säureadditionssalze mit anorganischen und organischen Säuren, und Verfahren zu ihrer Herstellung.

Die neuen Verbindungen und deren physiologisch verträgliche Säureadditionssalze weisen wertvolle pharmakologische Eigenschaften auf, insbesondere eine Wirkung auf den Blutdruck und auf die Kontraktilität des Herzmuskels sowie antithrombotische Wirkungen. Ein weiterer Gegenstand der vorliegenden Erfindung sind daher die neuen diese Verbindungen enthaltenden Arzneimittel, die zur Behandlung von Herzinsuffizienzen unterschiedlicher Genese, zur Behandlung und zur Prophylaxe thrombo-embolischer Erkrankungen, zur Prophylaxe der Arteriosklerose und zur Metastasenprophylaxe geeignet sind, und Verfahren zu ihrer Herstellung.

In der obigen allgemeinen Formel I bedeutet

R ein Wasserstoffatom oder eine Alkylgruppe,

Ar eine Gruppe der Formel

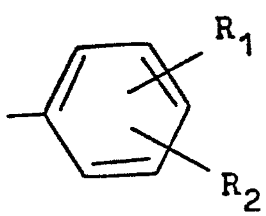

oder

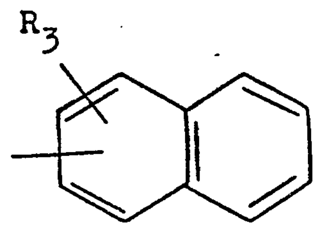

und

A und B zusammen mit den beiden dazwischen liegenden Kohlenstoffatomen eine Gruppe der Formel

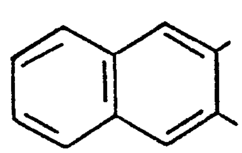

,

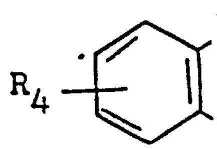

,

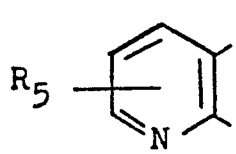

,

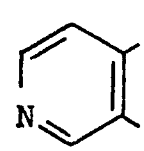

,

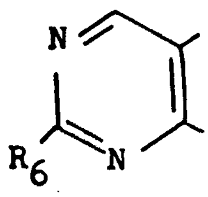

oder

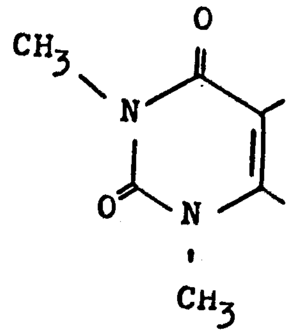

,

wobei

$R_1$ eine Alkylsulfenyl-, Alkylsulfinyl-, Alkylsulfonyl- oder Alkylsulfoximinogruppe, eine am Stickstoffatom durch eine Alkanoyl-, Alkylsulfonyl- oder Hydroxycarbonyl-alkylencarbonylgruppe substituierte Alkylsulfoximinogruppe, eine Ethoxy- oder n-Propoxygruppe, die jeweils endständig durch eine Alkylsulfenyl-, Alkylsulfinyl-, Alkylsulfonyl- oder Alkylsulfoximinogruppe substituiert ist, eine Alkoxycarbonylamino- oder N-Alkylaminocarbonyl-aminogruppe oder auch in 4-Stellung eine Hydroxy-, Phenylalkoxy-, Aminosulfonyl-, Alkylsulfonyloxy- oder Alkylsulfonylaminogruppe, wenn

a) A und B zusammen mit den beiden dazwischen liegenden Kohlenstoffatomen eine

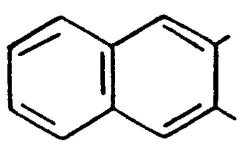

-Gruppe oder

b) $R_4$ eine Alkyl-, Alkanoyl-, N-Alkanoyl-aminomethyl-, Benzoyl-, Phenyl-methoxymethyl-, Aminosulfonyl-, N-Ethylaminocarbonyl-amino-, N-n-Propylaminocarbonyl-amino-, N-Isopropylaminocarbonyl-amino- oder N-Alkyl-N-alkylaminocarbonyl-aminogruppe oder

c) $R_6$ die Aminogruppe darstellt, wobei mindestens einer der Reste die unter a), b) und c) aufgeführten Bedeutungen darstellen muß,

$R_2$ ein Wasserstoffatom oder eine Alkoxygruppe,

$R_3$ eine Ethoxy- oder n-Propoxygruppe, die jeweils endständig durch eine Alkylsulfenyl-, Alkylsulfinyl-, Alkylsulfonyl- oder Alkylsulfoximinogruppe substituiert ist,

$R_4$ eine Alkyl-, Alkoxycarbonyl-, Alkanoyl-, N-Alkanoyl-aminomethyl-, Benzoyl-, Phenyl-methoxymethyl-, Aminocarbonyl-, Cyano-, Trifluormethyl-, Aminosulfonyl-, N-Alkylaminocarbonyl-amino- oder N-Alkyl-N-alkylaminocarbonyl-aminogruppe,

$R_5$ ein Wasserstoff- oder Halogenatom, eine Alkyl- oder Cyanogruppe und

$R_6$ ein Wasserstoffatom oder eine Aminogruppe unter der Voraussetzung bedeuten, daß

a) $R_1$ keine Alkylmercaptogruppe in 4-Stellung darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen,

b) $R_1$ keine Alkylsulfinylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

c) $R_1$ keine Alkylsulfonylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

d) $R_1$ keine Alkylsulfoximinogruppe in 4-Stellung darstellt, wenn $R_4$ in 5-Stellung eine Cyano- oder Carbamidogruppe und gleichzeitig $R_2$ in 2-Stellung eine Methoxy- oder Ethoxygruppe und R ein Wasserstoffatom darstellen, oder

e) $R_1$ in 4-Stellung keine Hydroxy- oder Phenylalkoxygruppe darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen,

wobei die vorstehend erwähnten Alkyl-, Alkylen-, Alkoxy- und Alkanoylteile jeweils 1 bis 3 Kohlenstoffatome enthalten können.

Für die bei der Definition der Reste R und $R_1$ bis $R_6$ eingangs erwähnten Bedeutungen kommt beispielsweise

für R die Bedeutung des Wasserstoffatoms, der Methyl-, Ethyl-, n-Propyl- oder Isopropylgruppe,

für $R_1$ die der Methylmercapto-, Ethylmercapto-, n-Propylmercaotp-, Methylsulfinyl-, Ethylsulfinyl-, Isopropylsulfinyl-, Methylsulfonyl-, Ethylsulfonyl-, n-Propylsulfonyl-, Methylsulfoximino-, Ethylsulfoximino-, Isopropylsulfoximino-, N-Acetyl-methylsulfoximino-, N-Propionyl-methylsulfoximino-, N-Methansulfonyl-methylsulfoximino-, N-Ethansulfonyl-methylsulfoximino-, N-Hydroxycarbonylmethylencarbonylmethylsulfoximino-, N-(2-Hydroxycarbonyl-äthylencarbonyl)-methylsulfoximino-, N-(3-Hydroxycarbonyl-propylencarbonyl)-methylsulfoximino-, N-Acetyl-ethylsulfoximino-, N-Methansulfonyl-ethylsulfoximino-, N-Acetyl-n-propylsulfoximino-, 2-Methylmercapto-ethoxy-, 2-Isopropylmercapto-ethyoxy-, 2-Methylsulfinyl-ethoxy-, 2-Methylsulfonyl-ethoxy-, 2-Methylsulfoximino-ethoxy-, 3-Methylmercapto-n-propoxy-, 3-Methylsulfinyl-n-propoxy-, 3-Methylsulfonyl-n-propoxy-, 3-Methylsulfoximino-n-propoxy-, Methoxycarbonylamino-, Ethoxycarbonylamino-, n-Propoxycarbonylamino-, N-Methylaminocarbonyl-amino-, N-Ethylaminocarbonyl-amino-, N-Isopropylaminocarbonyl-, Hydroxy-, Benzyloxy-, 1-Phenyl-ethoxy-, 2-Phenyl-ethoxy-, 3-Phenyl-propoxy-, Aminosulfonyl-, Methylsulfonyloxy-, Ethylsulfonyloxy-, n-Propylsulfonyloxy-, Methansulfonylamino-, Ethansulfonylamino- oder Propansulfonylaminogruppe,

für $R_2$ die des Wasserstoffatoms, der Methoxy-, Ethoxy-, n-Propoxy- oder Isopropoxygruppe,

für $R_3$ die 2-Methylmercapto-ethoxy-, 2-Ethylmercapto-ethoxy-, 2-n-Propylmercapto-ethoxy-, 2-Methylsulfinyl-ethoxy-, 2-Isopropylsulfinyl-ethoxy-, 2-Methylsulfonyl-ethoxy-, 2-Ethylsulfonyl-ethoxy-, 2-Methylsulfoximino-ethoxy-, 2-Ethylsulfoximino-ethoxy-, 2-n-Propylsulfoximino-ethoxy-, 3-Methylmercapto-propoxy-, 3-Ethylmercapto-propoxy-, 3-n-Propylmercapto-propoxy-, 3-Methylsulfinyl-propoxy-, 3-Isopropylsulfinyl-propoxy-, 3-Methylsulfonyl-propoxy-, 3-Ethylsulfonyl-propoxy-, 3-Methylsulfoximino-propoxy-, 3-Ethylsulfoximino-propoxy- oder 3-n-Propylsulfoximino-propoxygruppe,

für $R_4$ die der Methyl-, Ethyl-, n-Propyl-, Methoxycarbonyl-, Ethoxycarbonyl-, Isopropoxycarbonyl-, Acetyl-, Propionyl-, Benzoyl-, N-Acetyl-aminomethyl-, N-Propionyl-aminomethyl-, Phenyl-methoxymethyl-, Aminocarbonyl-, Cyano-, Trifluoromethyl-, Aminosulfonyl-, N-Methylaminocarbonyl-amino-, N-Ethylaminocarbonyl-amino-, N-n-Propylaminocarbonyl-amino-, N-Isopropylaminocarbonyl-amino-, N-Methyl-N-methylaminocarbonyl-amino-, N-Ethyl-N-ethylaminocarbonyl-amino- oder N-Methyl-N-ethylaminocarbonyl-aminogruppe,

für $R_5$ die des Wasserstoff-, Fluor-, Chlor- oder Bromatoms, der Cyano-, Methyl-, Ethyl-, n-Propyl- oder Isopropylgruppe und

für $R_6$ die des Wasserstoffatoms oder der Aminogruppe in Betracht.

Bevorzugte Verbindungen der obigen allgemeinen Formel I sind jedoch diejenigen, in denen

R ein Wasserstoffatom oder die Methylgruppe,

$R_1$ eine Methylsulfenyl-, Ethylsulfenyl-, Methylsulfinyl-, Ethylsulfinyl-, Methylsulfonyl-, Ethylsulfonyl-, Methylsulfoximino-, Ethylsulfoximino- oder n-Propylsulfoximinogruppe, eine am Stickstoffatom durch eine Acetyl-, Methansulfonyl- oder Hydroxycarbonyl-äthylencarbonylgruppe substituierte Methylsulfoximinogruppe, eine Ethoxygruppe, die endständig durch eine Methylsulfoximinogruppe substituiert ist, eine Methoxycarbonylamino- oder N-Methylaminocarbonyl-aminogruppe oder auch in 4-Stellung eine Hydroxy-, Benzyloxy-, Aminosulfonyl-, Methansulfonyloxy- oder Methansulfonylaminogruppe, wenn

a) A und B zusammen mit den beiden dazwischen liegenden Kohlenstoffatomen eine

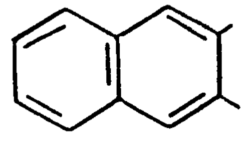

-Gruppe oder

b) $R_4$ eine Methyl-, Acetyl-, N-Acetyl-aminomethyl-, Benzoyl-, Phenyl-methoxymethyl-, Aminosulfonyl-, N-Ethylaminocarbonyl-amino-, N-Isopropylaminocarbonyl-amino- oder N-Methyl-N-methylaminocarbonyl-aminogruppe oder

c) $R_6$ die Aminogruppe darstellt, wobei mindestens einer der Reste die unter a), b) und c) aufgeführten Bedeutungen darstellen muß,

$R_2$ ein Wasserstoffatom, eine Methoxy- oder eine Ethoxygruppe,

$R_3$ eine Ethoxygruppe, die endständig durch eine Methylsulfenyl-, Methylsulfinyl-, Methylsulfonyl- oder Methylsulfoximinogruppe substituiert ist,

$R_4$ eine Methyl-, Methoxycarbonyl-, Acetyl-, N-Acetylaminomethyl-, Benzoyl-, Phenyl-methoxymethyl-, Aminocarbonyl-, Cyano-, Trifluoromethyl-, Aminosulfonyl-, N-Ethylaminocarbonyl-amino-, N-Isopropylaminocarbonyl-amino- oder N-Methyl-N-methylaminocarbonyl-aminogruppe,

$R_5$ ein Wasserstoff- oder Chloratom, eine Methyl- oder Cyanogruppe und

$R_6$ ein Wasserstoffatom oder eine Aminogruppe unter der Voraussetzung bedeuten, daß

a) $R_1$ keine Methyl- oder Ethylmercaptogruppe in 4-Stellung darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen,

b) $R_1$ keine Methyl- oder Ethylsulfinylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

c) $R_1$ keine Methyl- oder Ethylsulfonylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

d) $R_1$ keine Methyl- oder Ethylsulfoximinogruppe in 4-Stellung darstellt, wenn $R_4$ in 5-Stellung eine Cyano- oder Carbamidogruppe und gleichzeitig $R_2$ in 2-Stellung eine Methoxy- oder Ethoxygruppe und R ein Wasserstoffatom darstellen, oder

e) $R_1$ in 4-Stellung keine Hydroxy- oder Benzyloxygruppe darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen, sowie die Verbindungen

5-Cyano-2-(4'-methansulfonylamino-2'-methoxy-phenyl)-benzimidazol,

4-Methyl-2-(4'-methansulfonyloxy-2'-methoxy-phenyl)-benzimidazol,

4-Cyano-2-(4'-methansulfonyloxy-2'-methoxy-phenyl)-benzimidazol,

4-Aminocarbonyl-2-(4'-methansulfonyloxy-2'-methoxy-phenyl)-benzimidazol und

4-Methoxycarbonyl-2-(4'-methansulfonyloxy-2'-methoxy-phenyl)-benzimidazol,

deren Tautomere und deren Säureadditionssalze, insbesondere deren physiologisch verträgliche Säureadditionssalze.

Besonders bevorzugte Verbindungen der allgemeinen Formel I sind jedoch

2-[2'-Methoxy-4'-(N-acetyl-methylsulfoximino-phenyl)]-5-cyano-benzimidazol,

2-(2'-Methoxy-4'-methylsulfinyl-phenyl)-5-acetyl-benzimidazol,

2-(2'-Methoxy-4'-methylsulfonyl-phenyl)-5-acetyl-benzimidazol,

5-Cyano-2-(4'-methansulfonylamino-2'-methoxyphenyl)-benzimidazol,

2-(2'-Methoxy-4'-methoxycarbonylamino-phenyl)-imidazo[4,5-c]-pyridin,

6-Cyano-2-(2'-methoxy-4'-methylmercapto-phenyl)-imidazo-[4,5-b]pyridin,

2-Amino-8-(2'-methoxy-4'-methylsulfinyl-phenyl)-purin,

2-(2'-Methoxy-4'-methylsulfoximino-phenyl)-1H-imidazo[4,5-c]-pyridin,

5-Aminocarbonyl-2-(4'-methansulfonyloxy-2'-methoxy-phenyl)-benzimidazol und

5-Acetyl-2-(4'-methansulfonyloxy-2'-methoxy-phenyl)-benzimidazol, deren Tautomere und deren Säureadditionssalze, insbesondere deren physiologisch verträgliche Säureadditionssalze.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

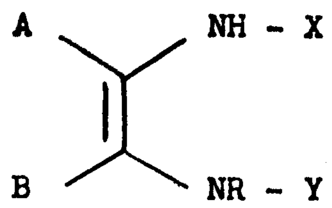

(II)

in der
A, B und R wie eingangs definiert sind,
einer der Reste X oder Y ein Wasserstoffatom und der andere der beiden Reste X und Y oder beide Reste X und Y eine Gruppe der Formel

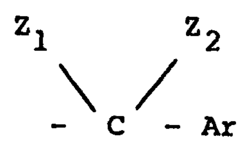

darstellen, in der

Ar wie eingangs definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der $R_1$ eine Alkylsulfinyl-, Alkylsulfonyl- oder Alkylsulfoximinogruppe, eine am Stickstoffatom durch eine Alkanoyl-, Alkylsulfonyl- oder Hydroxycarbonyl-alkylencarbonylgruppe substituierte Alkylsulfoximinogruppe, eine Ethoxy- oder n-Propoxygruppe, die endständig durch eine Alkylsulfinyl-, Alkylsulfonyl- oder Alkylsulfoximinogruppe substituiert ist, oder $R_3$ eine Ethoxy- oder n-Propoxygruppe, die endständig durch eine Alkylsulfinyl-, Alkylsulfonyl- oder Alkylsulfoximinogruppe substituiert ist, darstellen:

Oxidation einer Verbindung der allgemeinen Formel

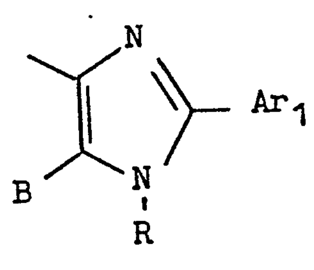

,(III)

in der

A, B und R wie eingangs definiert sind und

$Ar_1$ die für Ar eingangs erwähnten Bedeutungen besitzt, wobei jedoch $R_1$ eine Alkylsulfenyl-, Alkylsulfinyl- oder Alkylsulfiminogruppe, eine am Stickstoffatom durch eine Alkanoyl-, Alkylsulfonyl- oder Hydroxycarbonyl-alkylencarbonylgruppe substituierte Alkylsulfiminogruppe, eine Ethoxy-

oder n-Propoxygruppe, die endständig durch eine Alkylsulfenyl-, Alkylsulfinyl- oder Alkylsulfiminogruppe substituiert ist, oder $R_3$ eine Ethoxy- oder n-Propoxygruppe, die endständig durch eine Alkylsulfenyl-, Alkylsulfinyl- oder Alkylsulfiminogruppe substituiert ist, darstellen muß.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Alkylsulfinyl- oder Sulfoximinoverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure, mit N-Brom-succinimid in Äthanol,
mit tert.Butyl-hyprochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Alkylsulfonylverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

c) Zur Herstellung von Verbindungen der allgemeinen Formel I, in der $R_1$ eine Alkylsulfoximinogruppe, eine Ethoxy- oder n-Propoxygruppe, die endständig durch eine Alkylsulfoximinogruppe substituiert ist, oder $R_3$ eine Ethoxy- oder n-Propoxygruppe, die endständig durch eine Alkylsulfoximinogruppe substituiert ist, darstellt:

Umsetzung eines Sulfoxids der allgemeinen Formel

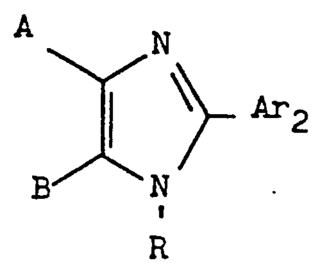
,(IV)

in der
A, B und R wie eingangs definiert sind und
$Ar_2$ die für Ar eingangs erwähnten Bedeutungen besitzt, wobei jedoch $R_1$ eine Alkylsulfinylgruppe, eine Ethoxy- oder n-Propoxygruppe, die endständig durch eine Alkylsulfinyl-

gruppe substituiert ist, oder $R_3$ eine Ethoxy- oder n-Propoxygruppe, die endständig durch eine Alkylsulfinylgruppe substituiert ist, darstellen muß, mit gegebenenfalls im Reaktionsgemisch gebildeter Stickstoffwasserstoffsäure.

Die Umsetzung wird zweckmäßigerweise in einem Lösungsmittel oder Lösungsmittelgemisch wie Methylenchlorid, Dimethylformamid oder Tetrahydrofuran bei Temperaturen zwischen 0 und 40°C, vorzugsweise bei Temperaturen zwischen 10 und 35°C, durchgeführt. Besonders vorteilhaft wird die Umsetzung mit einem Alkaliazid, z.B. Natriumazid, und Polyphosphorsäure als Lösungsmittel durchgeführt.

d) Zur Herstellung von Verbindungen der allgemeinen Formel I, in der $R_1$ eine Alkylsulfoximinogruppe, eine Ethoxy- oder n-Propoxygruppe, die endständig durch eine Alkylsulfoximinogruppe substituiert ist, oder $R_3$ eine Ethoxy- oder n-Propoxygruppe, die endständig durch eine Alkylsulfoximinogruppe substituiert ist, darstellt:

Umsetzung eines Sulfoxids der allgemeinen Formel

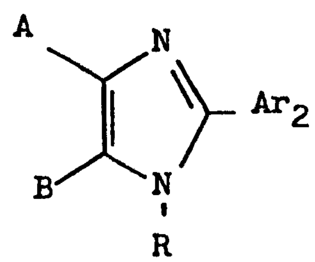

,(IV)

in der

A, B und R wie eingangs definiert sind und

$Ar_2$ die für Ar eingangs erwähnten Bedeutungen besitzt, wobei jedoch $R_1$ eine Alkylsulfinylgruppe, eine Ethoxy- oder n-Propoxygruppe, die endständig durch eine Alkylsulfinylgruppe substituiert ist, oder $R_3$ eine Ethoxy- oder n-Propoxygruppe, die endständig durch eine Alkylsulfinylgruppe substituiert ist, darstellen muß, mit einer gegebenfalls im Reaktionsgemisch hergestellten Verbindung der allgemeinen Formel

$$H_2N - O - U - R_7 \quad ,(V)$$

in der
X eine Carbonyl- oder Sulfonylgruppe und
$R_7$ eine in o-Stellung disubstituierte Arylgruppe wie eine 2,4,6-Trimethylphenyl- oder 2,4,6-Triisopropylphenylgruppe darstellen.

Die Umsetzung wird zweckmäßigerweise in einem Lösungsmittel oder Lösungsmittelgemisch wie Methlenchlorid, Chloroform, Dimethylformamid, Tetrahydrofuran oder Dioxan bei Temperaturen zwischen 0 und 50°C, vorzugsweise jedoch bei Temperaturen zwischen 5 und 40°C, und gegebenenfalls in Gegenwart einer katalytischen Menge einer Säure wie p-Toluolsulfonsäure durchgeführt. Besonders vorteilhaft wird die Umsetzung jedoch in der Weise durchgeführt, daß eine Verbindung der allgemeinen Formel V ohne ihre vorherige Isolierung eingesetzt bzw. im Reaktionsgemisch hergestellt wird.

e) Zur Herstellung einer Verbindung der allgemeinen Formel I, in der $R_1$ eine am Stickstoffatom durch eine Alkanoyl-, Alkylsulfonyl- oder Hydroxycarbonyl-alkylencarbonylgruppe substituierte Alkylsulfoximinogruppe darstellt:

Acylierung einer Verbindung der allgemeinen Formel

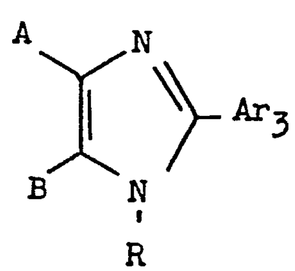

,(VI)

in der
A, B und R wie eingangs definiert sind und
$Ar_3$ die für Ar eingangs erwähnten Bedeutungen besitzt, wobei jedoch $R_1$ eine Alkylsulfoximinogruppe darstellen muß, mit einer Verbindung der allgemeinen Formel

$$R_8 - V \qquad ,(VII)$$

in der
$R_8$ eine Alkanoyl-, Alkylsulfonyl- oder Hydroxycarbonylalkylencarbonylgruppe und
V eine nukleofuge Austrittsgruppe wie ein Halogenatom oder eine Alkanoyloxygruppe darstellen, oder deren Ester und Anhydride.

Die Umsetzung wird zweckmäßigerweise in einem Lösungsmittel oder Lösungmittelgemisch wie Wasser, Methylenchlorid, Chloroform, Äther, Tetrahydrofuran, Dioxan oder Dimethylformamid mit einer entsprechenden Verbindung in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

f) Zur Herstellung von Verbindungen der allgemeinen Formel I, in der $R_1$ eine Alkylsulfonyloxy-, Alkylsulfonylamino-, Alkoxycarbonylamino- oder N-Alkylaminocarbonyl-aminogruppe oder $R_4$ eine Alkanoylaminomethyl-, N-Ethylaminocarbonylamino-, N-n-Propylaminocarbonyl-amino-, N-Isopropylaminocarbonyl-amino- oder N-Alkyl-N-alkylaminocarbonyl-aminogruppe darstellt:

Acylierung einer Verbindung der allgemeinen Formel

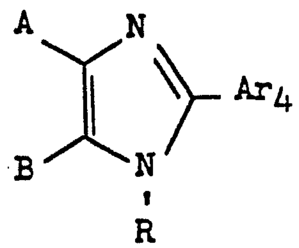

,(VIII)

in der
A, B und R wie eingangs definiert sind und
$Ar_4$ die für Ar eingangs erwähnten Bedeutungen besitzt, wobei jedoch $R_1$ eine Hydroxy- oder Aminogruppe oder $R_4$ eine Aminomethyl- oder Aminogruppe darstellen muß, mit einer Verbindung der allgemeinen Formel

$$R_9 - W \qquad ,(IX)$$

in der
$R_9$ eine Alkylgruppe mit 1 bis 3 Kohlenstoffatomen und
W eine O=N=C- oder Halogensulfonylgruppe wie die Chlor- oder Bromsulfonylgruppe oder

$R_9$ eine Methyl- oder Ethylgruppe und
W eine -CO-V-Gruppe darstellen, wobei V eine nucleofuge Austrittsgruppe wie ein Halogenatom oder eine Alkanoyloxygruppe darstellt.

Die Umsetzung wird zweckmäßigerweise in einem Lösungsmittel oder Lösungsmittelgemisch wie Methylenchlorid, Chloroform, Tetrachlorkohlenstoff, Ether, Tetrahydrofuran, Dioxan, Benzol, Toluol, Acetonitril oder Dimethylformamid, gegebenenfalls auch in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol, und gegebenenfalls in Gegenwart einer anorganischen Base wie Natriumcarbonat oder einer tertiären organischen Base wie Triethylamin oder Pyridin, wobei die beiden letzteren auch gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden.

g) Zur Herstellung von Verbindungen der allgemeinen Formel I, in der R eine Alkylgruppe darstellt:

Alkylierung einer Verbindung der allgemeinen Formel

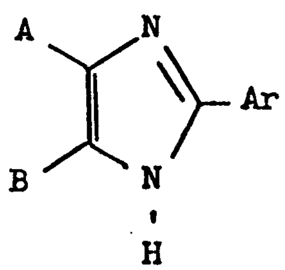

,(X)

in der
A, B und Ar wie eingangs definiert sind.

Die Umsetzung wird mit einem entsprechenden Alkylierungsmittel wie Methyljodid, Ethyljodid, Diethylsulfat, Dimethylsulfat oder n-Propylbromid zweckmäßigerweise in einem Lösungsmittel oder Lösungsmittelgemisch wie Methylenchlorid, Ether, Tetrahydrofuran, Dioxan, Wasser oder Benzol gegebenenfalls in Gegenwart eines säurebindenden Mittels wie Natriumcarbonat, Natriumhydroxid, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Erhält man erfindungsgemäß eine Verbindung der allgemeinen Formel I, in der $R_1$ eine Benzyloxygruppe darstellt, so kann diese mittels Entbenzylierung in die entsprechende Hydroxyverbindung übergeführt werden, und/oder

eine erhaltene Verbindung der allgemeinen Formel I, in der $R_4$ eine Cyangruppe darstellt, so kann diese mittels Hydrolyse in die entsprechende Aminocarbonylverbindung übergeführt werden.

Die nachträgliche Entbenzylierung wird vorzugsweise in einem Lösungsmittel wie Wasser, Wasser/Ethanol, Methanol, Eisessig, Essigsäureethylester oder Dimethylformamid zweckmäßigerweise mit Wasserstoff in Gegenwart eines Hydrierungskatalysators wie Raney-Nickel, Platin oder Palladium/Kohle bei Temperaturen zwichen 0 und 50°C, vorzugsweise jedoch bei Raumtemperatur, durchgeführt.

Die nachträgliche Hydrolyse wird in Gegenwart einer anorganischen Base mit Wasserstoffperoxid, z.B. mit 2 n Natronlauge oder Kalilauge/Wasserstoffperoxid, bei Temperaturen zwischen 0 und 50°C, vorzugsweise jedoch bei Raumtemperatur, durchgeführt.

Die erfindungsgemäß erhaltenen neuen Verbindungen können anschließend gewünschtenfalls in ihre Säureadditionssalze übergeführt werden. Die so erhaltenen Verbindungen lassen sich ferner zur pharmazeutischen Anwendung in ihre physiologisch verträglichen Säureadditionssalze überführen. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis X sind teilweise literaturbekannt bzw. man erhält sie nach literaturbekannten Verfahren. so erhält man beispielsweise die als Ausgangsstoffe verwendeten Verbindungen der allgemeinen Formel II durch Acylierung der entsprechenden o-Diaminoverbindungen bzw. durch Reduktion

der entsprechenden Acylamino-nitro-Verbindungen sowie die Verbindungen der allgemeinen Formeln III, IV, VI, VII oder X durch Ringschluß einer entsprechenden o-Acylamino-aminoverbindung (siehe BE-PS 810.545 und EP-A-0.024.290) und gegebenenfalls anschließende Entbenzylierung.

Wie bereits eingangs erwähnt weisen die neuen Verbindungen der allgemeinen Formel I, deren 1H Tautomere und deren physiologisch verträgliche Säureadditionssalze bei einer langen Wirkungsdauer überlegene pharmakologische Eigenschaften auf, insbesondere eine blutdrucksenkende, positiv-inotrope und/ oder antithrombotische Wirkung.

Beispielsweise wurden die Verbindungen

A = 2-[2'-Methoxy-4'-(N-acetyl-methylsulfoximino-phenyl)]-5-cyano-benzimidazol,

B = 2-(2'-Methoxy-4'-methylsulfinyl-phenyl)-5-acetyl-benzimidazol,

C = 2-(2'-Methoxy-4'-methylsulfonyl-phenyl)-5-acetyl-benzimidazol,

D = 5-Cyano-2-(4'-methansulfonylamino-2'-methoxy-phenyl)-benzimidazol und

E = 2-(2'-Methoxy-4'-methoxycarbonylamino-phenyl)-imidazo-[4,5-c]pyridin

auf ihre biologischen Eigenschaften wie folgt untersucht:

1. Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter $dp/dt_{max}$ mittels eines Analogdifferenzierers gewonnen. Die zu untersuchenden Substanzen wurden in eine Vena femoralis injiziert. Als Lösungsmittel diente Polydiol 200. Jede Substanz wurde an mindestens 2 Katzen geprüft.

Die nachfolgende Tabelle enthält die gefundenen Mittelwerte:

| Substanz | Dosis mg/kg i.v. | Zunahme von dp/dt in % | Blutdruck senkung in mm Hg |
|---|---|---|---|
| A | 1 | + 33 | -25/-20 |
| B | 1 | + 44 | -29/-28 |
| C | 1 | + 46 | -26/-24 |
| D | 0,6 | + 71 | -40/-27 |
| E | 2 | + 62 | -17/-12 |

2. Antithrombotische Wirkung

Methodik

Die Thrombozytenaggregation wird nach der Methode von BORN und CROSS (J. Physiol. 170, 397 (1964)) in plättchenreichem

Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ berechnet, die sich auf eine 50%ige Änderung der "optical density" im Sinne einer Aggregationshemmung bezieht.

Die nachfolgende Tabelle enthält die gefundenen Ergebnisse:

| Substanz | $EC_{50}$ [µMol/l] |
|---|---|
| A | 5,3 |
| B | 8,2 |
| C | 8,0 |

Die neuen Verbindungen sind gut verträglich, so konnte bei der Untersuchung der Substanzen keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und durch die Blutdrucksenkung die Entleerung des Herzens erleichtern, zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe.

Die zur Erzielung einer entsprechenden Wirkung erforderliche Dosierung beträgt zweckmäßigerweise zwei- bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel 1

1(3)-Methyl-2-(2'-methoxy-4'-methylmercapto-phenyl)-5-cyano-benzimidazol

1,0 g (0,003386 Mol) 2-(2'-Methoxy-4'-methylmercapto-phenyl)-5-cyano-benzimidazol, gelöst in 15 ml Dimethylformamid, werden mit 1,90 g (5 x 0,003386 Mol) Kalium-tert.butylat und anschließend unter Rühren mit 1,6 ml (5 x 0,003386 Mol) Dimethylsulfat versetzt. Nach 30 Minuten Rühren verteilt man 4 mal zwischen 20 ml Wasser und je 20 ml Essigester. Die vereinigten Esssigesterextrakte werden nach Schütteln mit 20 ml gesättigter Kochsalzlösung über Magnesiumsulfat getrocknet und zur Trockne eingedampft. Man erhält ein Öl, welches teilweise kristallisiert.
Ausbeute: 1,05 g (60 % der Theorie),
Das Dünnschichtchromatogramm (Kieselgel; Methylenchlorid/Essigester = 1:1) zeigt ein Isomer bei $R_f$= 0,45 und ein Isomer bei $R_f$= 0,40. Durch Säulenchromatographie an Kieselgel mit Methylenchlorid/Essigester wird das Isomer mit $R_f$= 0,45 rein abgetrennt.
Schmelzpunkt: 212-214°C.

Beispiel 2

1(3)-Methyl-2-(2'-methoxy-4'-methylsulfonyl-phenyl)-5-cyano-benzimidazol

Hergestellt durch Oxidation von 1(3)-Methyl-2-(2'-methoxy-4'-methylmercapto-phenyl)-5-cyano-benzimidazol in Ameisensäure mit der 3-fachen molaren Menge Wasserstoffperoxid. Man erhält weiße Kristalle in einer Ausbeute von 80 % der Theorie, die in einem Interval von 194-220°C schmelzen (Isomerengemisch).

Beispiel 3

1(3)-Methyl-2-(2'-methoxy-4'-methylsulfinyl-phenyl)-5-cyano-benzimidazol

485 mg (0,001568 Mol) 1(3)-Methyl-2-(2'-methoxy-4'-methylmercapto-phenyl)-5-cyano-benzimidazol werden in 5 ml Eisessig mit 0,12 ml Wasserstoffperoxid (399 mg/ml; entsprechend 0,9 x 0,001568 Mol) bei Raumtemperatur 65 Stunden lang stehen gelassen. Man erhält weiße Kristalle.
Schmelzintervall: 168-175°C (Isomerengemisch).
Ausbeute: 330 mg (64,7 % der Theorie).

Beispiel 4

1(3)-Methyl-2-(2'-methoxy-4'-methylsulfoximino-phenyl)-5-cyano-benzimidazol

1,048 g (0,00322 Mol) 1(3)-Methyl-2-(2'-methoxy-4'-methylsulfinyl-phenyl)-5-cyano-benzimidazol werden in 7 ml Dimethylformamid zusammen mit 2,3 g (2,5 x 0,00322 Mol) O-Mesitylensulfonyl-acethydroxamsäure-ethylester und 2,87 g (4,5 x 0,00322 Mol) 4-Toluolsulfonsäurehydrat unter Rühren gelöst und 48 Stunden bei Raumtemperatur stehen gelassen. Man stellt mit 2n Natronlauge unter Kühlen alkalisch und extrahiert erschöpfend mit einem Gemisch aus Chloroform/Äthanol = 9:1. Nach dem Abdampfen des Lösungsmittelgemisches chromatographiert man an einer Kieselgelsäule mit Äthylenchlorid/Äthanol = 4:1. Man erhält weiße Kristalle.
Schmelzintervall: 207-218°C,
Ausbeute: 0,450 g (41 % der Theorie).

Beispiel 5

2-[2'-Methoxy-4'-(N-methansulfonyl-methylsulfoximino)-phenyl]-5-cyano-benzimidazol

Hergestellt aus 2-(2'-Methoxy-4'-methylsulfoximino-phenyl)-5-cyano-benzimidazol und Methansulfonylchlorid in Pyridin.
Schmelzpunkt: 270-273°C,
Ausbeute: 62 % der Theorie.

Beispiel 6

2-[2'-Methoxy-4'-(N-acetyl-methylsulfoximino)-phenyl]-5-cyano-benzimidazol

Hergestellt aus 2-(2'-Methoxy-4'-methylsulfoximino-phenyl)-5-cyano-benzimidazol und Acetanhydrid.
Schmelzpunkt: 212,5-214°C,
Ausbeute: 89,6 % der Theorie.

Beispiel 7

2-(2'-Methoxy-4'-methylmercapto-phenyl)-5-trifluormethyl-benzimidazol

5,0 g (0,02 Mol) 2-Amino-4'-trifluormethylanilin und 4,0 g (0,02 Mol) 2-Methoxy-4-methylmercapto-benzoesäure werden in 90 ml Phosphoroxychlorid 2 Stunden lang zum Sieden erhitzt. Man entfernt im Rotationsverdampfer im Vakuum das überschüssige Phosphoroxychlorid, versetzt den Rückstand mit

Eiswasser und neutralisiert mit Natriumhydrogenkarbonat. Das ausgefallene Reaktionsprodukt wird abgesaugt und aus Äthanol umkristallisiert.
Schmelzpunkt: 144-147°C,
Ausbeute: 4,78 g (70,4 % der Theorie).

Beispiel 8

2-(2'-Methoxy-4'-methylsulfinyl-phenyl)-5-trifluormethyl-benzimidazol

Hergestellt analog Beispiel 3 aus 2-(2'-Methoxy-4'-methylmercapto-phenyl)-5-trifluormethyl-benzimidazol und Wasserstoffperoxid.
Schmelzpunkt: 65°C (sintern),
Zersetzung bei 120°C,
Ausbeute: 99,1 % der Theorie.

Beispiel 9

2-(2'-Methoxy-4'-methylsulfonyl-phenyl)-5-trifluormethyl-benzimidazol

Hergestellt analog Beispiel 2 aus 2-(2'-Methoxy-4'-methylmercapto-phenyl)-5-trifluormethyl-benzimidazol und Wasserstoffperoxid.
Schmelzpunkt: 217-220°C,
Ausbeute: 84 % der Theorie.

## Beispiel 10

**2-(2'-Methoxy-4'-methylsulfoximino-phenyl)-5-trifluormethyl-benzimidazol**

Hergestellt analog Beispiel 4 aus 2-(2'-Methoxy-4'-methylsulfinyl-phenyl)-5-trifluormethyl-benzimidazol und O-Mesitylensulfonyl-acethydroxamsäure-ethylester.
Schmelzpunkt: 154°C,
Ausbeute: 63 % der Theorie.

## Beispiel 11

**2-(4'-Methylmercapto-phenyl)-5-trifluormethyl-benzimidazol**

Hergestellt analog Beispiel 7 aus 2-Amino-4-trifluormethylanilin und 4-Methylmercapto-benzoesäure.
Schmelzpunkt: 162-164°C,
Ausbeute: 69 % der Theorie.

## Beispiel 12

**2-(4'-Methylsulfinyl-phenyl)-5-trifluormethyl-benzimidazol**

Hergestellt analog Beispiel 3 aus 2-(4'-Methylmercapto-phenyl)-5-trifluormethyl-benzimidazol und Wasserstoffperoxid.
Schmelzpunkt: 216-219°C,
Ausbeute 51 % der Theorie.

Beispiel 13

2-(4'-Methylsulfoximino-phenyl)-5-trifluormethyl-benzimidazol

Hergestellt analog Beispiel 4 aus 2-(4'-Methylsulfinyl-phenyl)-5-trifluormethyl-benzimidazol und O-Mesitylensulfonyl-acethydroxamsäure-ethylester.
Schmelzpunkt: 112-117°C,
Ausbeute: 56 % der Theorie.

Beispiel 14

2-(2'-Methoxy-4'-methylsulfinyl-phenyl)-5-methoxycarbonyl-benzimidazol

Hergestellt analog Beispiel 3 aus 2-(2'-Methoxy-4'-methylmercapto-phenyl)-5-methoxycarbonyl-benzimidazol und Wasserstoffperoxid.
Schmelzpunkt: 199-200°C,
Ausbeute: 88 % der Theorie.

Beispiel 15

2-(2'-Methoxy-4'-methylsulfonyl-phenyl)-5-methoxycarbonyl-benzimidazol

Hergestellt analog Beispiel 2 aus 2-(2'-Methoxy-4'-methylmercapto-phenyl)-5-methoxycarbonyl-benzimidazol und Wasserstoffperoxid.
Schmelzpunkt: 135°C,
Ausbeute: 87 % der Theorie.

**Beispiel 16**

**2-(2'-Methoxy-4'-methylsulfoximino-phenyl)-5-methoxycarbonyl-benzimidazol**

Hergestellt analog Beispiel 4 aus 2-(2'-Methoxy-4'-methylsulfinyl-phenyl)-5-methoxycarbonyl-benzimidazol und O-Mesitylensulfonyl-acethydroxamsäure-ethylester.
Schmelzpunkt: 214-216°C,
Ausbeute: 64 % der Theorie.

**Beispiel 17**

**2-(4'-Methylmercapto-phenyl)-5-cyano-benzimidazol**

Hergestellt analog Beispiel 7 aus 3,4-Diamino-benzonitril und 4-Methylmercapto-benzoesäure.
Schmelzpunkt: 213-215°C,
Ausbeute: 40 % der Theorie.

**Beispiel 18**

**2-(4'-Methylsulfinyl-phenyl)-5-cyano-benzimidazol**

Hergestellt analog Beispiel 3 aus 2-(4'-Methylmercapto-phenyl)-5-cyano-benzimidazol und Wasserstoffperoxid.
Schmelzpunkt: 266-268°C,
Ausbeute: 98 % der Theorie.

Beispiel 19

2-(4'-Methylsulfonyl-phenyl)-5-cyano-benzimidazol

Hergestellt analog Beispiel 2 aus 2-(4'-Methylmercapto-phenyl)-5-cyano-benzimidazol und Wasserstoffperoxid.
Schmelzpunkt: 271-273°C,
Ausbeute: 55 % der Theorie.

Beispiel 20

2-(4'-Methylsulfoximino-phenyl)-5-cyano-benzimidazol

Hergestellt analog Beispiel 4 aus 2-(4'-Methylsulfinyl-phenyl)-5-cyano-benzimidazol und O-Mesitylensulfonyl-acet-hydroxamsäure-ethylester.
Schmelzpunkt: 272-274°C,
Ausbeute: 78 % der Theorie.

Beispiel 21

2-[2'-Methoxy-4'-(N-3-carboxypropionyl-methylsulfoximino)]-5-cyano-benzimidazol

Hergestellt aus 2-(2'-Methoxy-4'-methylsulfoximino-phenyl)-5-cyano-benzimidazol und Bernsteinsäureanhydrid in siedendem Pyridin.
Schmelzpunkt: 265-268°C,
Ausbeute: 92 % der Theorie.

**Beispiel 22**

**2-(2'-Methoxy-5'-methylmercapto-phenyl)-5-cyano-benzimidazol**

a) 6,52 g 2-Methoxy-5-methylmercapto)-benz-(2-nitro-4-cyano)-anilid (hergestellt aus 2-Nitro-5-cyano-anilin und 2-Methoxy-5-methylmercapto-benzoylchlorid) werden in 70 ml Äthanol suspendiert, mit einer Lösung von 16,5 g Natriumdithionit in 70 ml Wasser versetzt und auf dem Dampfbad 30 Minuten lang erhitzt. Nach dem Einengen im Vakuum wird der erhaltene Rückstand mit wenig Wasser verrieben und abgesaugt. Das erhaltene 2-(2'-Methoxy-5-methylmercapto-phenyl)-benz-(2-amino-4-cyano)-anilid wird noch feucht in die nachfolgende Reaktion eingesetzt.

b) Das unter a) erhaltene Produkt wird in 15 ml Eisessig 2 Stunden lang zum Sieden erhitzt. Man destilliert im Vakuum den Eisessig ab und verteilt den Rückstand zwischen Essigester und kalter Sodalösung. Die Essigesterphase wird über Magnesiumsulfat getrocknet und eingeengt, wobei man leicht gelbe Kristalle erhält.
Schmelzpunkt: 171-173°C,
Ausbeute: 3,04 g (54 % der Theorie).

**Beispiel 23**

**2-(2'-Methoxy-5'-methylsulfinyl-phenyl)-5-cyano-benzimidazol**

Hergestellt analog Beispiel 3 aus 2-(2'-Methoxy-5'-methylmercapto-phenyl)-5-cyano-benzimidazol und Wasserstoffperoxid.
Schmelzpunkt: 215-217°C,
Ausbeute: 75 % der Theorie.

Beispiel 24

2-(2'-Methoxy-5'-methylsulfonyl-phenyl)-5-cyano-benzimidazol

Hergestellt analog Beispiel 2 aus 2-(2'-Methoxy-5'-methylmercapto-phenyl)-5-cyano-benzimidazol und Wasserstoffperoxid.
Schmelzpunkt: 276-278°C,
Ausbeute: 88 % der Theorie.

Beispiel 25

2-(2'-Methoxy-5'-methylsulfoximino-phenyl)-5-cyano-benzimidazol

Hergestellt analog Beispiel 4 aus 2-(2'-Methoxy-5'-methylsulfinyl-phenyl)-5-cyano-benzimidazol und O-Mesitylensulfonyl-acethydroxamsäure-ethylester.
Schmelzpunkt: 284-285°C,
Ausbeute: 84 % der Theorie.

Beispiel 26

2-(2'-Methoxy-4'-ethylmercapto-phenyl)-5-cyano-benzimidazol

Hergestellt analog Beispiel 22 aus 2-(2'-Methoxy-4'-ethylmercapto)-benz-(2-nitro-4-cyano)-anilid durch Reduktion mit Natriumdithionit und nachfolgende Cyclisierung.
Schmelzpunkt: 161-163°C,
Ausbeute: 85 % der Theorie.

**Beispiel 27**

**2-(2'-Methoxy-4'-ethylsulfinyl-phenyl)-5-cyano-benzimidazol**

Hergestellt analog Beispiel 3 aus 2-(2'-Methoxy-4'-ethylmercapto-phenyl)-5-cyano-benzimidazol und Wasserstoffperoxid.
Schmelzpunkt: 234-236°C,
Ausbeute: 98 % der Theorie.

**Beispiel 28**

**2-(2'-Methoxy-4'-ethylsulfonyl-phenyl)-5-cyano-benzimidazol**

Hergestellt analog Beispiel 2 aus 2-(2'-Methoxy-4'-ethylmercapto-phenyl)-5-cyano-benzimidazol und Wasserstoffperoxid.
Schmelzpunkt: 208-209°C,
Ausbeute: 85 % der Theorie.

**Beispiel 29**

**2-(2'-Methoxy-4'-ethylsulfoximino-phenyl)-5-cyano-benzimidazol**

Hergestellt analog Beispiel 4 aus 2-(2'-Methoxy-4'-ethylsulfinyl-phenyl)-5-cyano-benzimidazol und O-Mesitylensulfonyl-acethydroxamsäure-ethylester.
Schmelzpunkt: 252-254°C,
Ausbeute: 85 % der Theorie.

Beispiel 30

2-[2'-(2-Methylmercapto-ethoxy)-naphthyl-3]-5-cyano-benzimid-azol

Hergestellt analog Beispiel 7 aus 2-Amino-4-cyano-anilin und 2-(2-Methylmercapto-ethoxy)-3-naphtoesäure durch Sieden in Phosphoroxychlorid.
Schmelzpunkt: 186-188°C,
Ausbeute: 65 % der Theorie.

Beispiel 31

2-[2'-(2-Methylsulfinyl-ethoxy)-naphthyl-3]-5-cyano-benzimidazol

Hergestellt analog Beispiel 3 aus 2-[2'-(2-Methylmercapto-ethoxy)-naphthyl-3]-5-cyano-benzimidazol und Wasserstoffperoxid.
Schmelzpunkt: 207-208°C,
Ausbeute: 58 % der Theorie.

Beispiel 32

2-[2'-(2-Methylsulfonyl-ethoxy)-naphthyl-3]-5-cyano-benzimidazol

Hergestellt analog Beispiel 2 aus 2-[2'-(2-Methylmercapto-ethoxy)-naphthyl-3]-5-cyano-benzimidazol und Wasserstoffperoxid.
Schmelzpunkt: 257-259°C,
Ausbeute: 72 % der Theorie.

Beispiel 33

2-[2'-(2-Methylsulfoximino-ethoxy)-naphthyl-3)-5-cyano-benzimidazol

Hergestellt analog Beispiel 4 aus 2-[2'-(2-Methylsulfinyl-ethoxy)-naphthyl-3]-5-cyano-benzimidazol und O-Mesitylensulfonyl-acethydroxamsäure-ethylester.
Schmelzpunkt: 192-194°C,
Ausbeute: 52 % der Theorie.

Beispiel 34

2-(2'-Methoxy-4'-amidosulfonyl-phenyl)-5-acetyl-benzimidazol

a) 16 g 2-Methoxy-4-amidosulfonyl-benzoesäure werden mit 80 ml Thionylchlorid und 10 ml Dimethylformamid in 150 ml absolutem Toluol 30 Minuten lang zum Sieden erhitzt und dann wird im Vakuum zur Trockne eingedampft. Das erhaltene rohe, kristalline 2-Methoxy-4-(dimethylamino-formimido-sulfonyl)-benzoylchlorid wird in der nachfolgenden Reaktion unmittelbar eingesetzt.
Ausbeute: 21,5 g (100 % der Theorie).

b) 17,2 g 2-Methoxy-4-(dimethylamino-formimido-sulfonyl)-benzoylchlorid werden mit 12,4 g 3-Nitro-4-amino-acetophenon in 150 ml absolutem Toluol 2 Stunden lang zum Sieden am Rückfluß erhitzt. Hierbei trübt sich die anfangs klare Lösung durch auskristallisierendes 2-Methoxy-4-(dimethylamino-formimido-sulfonyl)-benz-(2-nitro-4-acetyl)-anilid,

welches nach dem Erkalten abgesaugt, mit wenig Methylethylketon gewaschen und getrocknet wird.
Schmelzpunkt: 230-235°C,
Ausbeute: 20,8 g (77 % der Theorie).

c) 19 g 2-Methoxy-4-(dimethylamino-formimido-sulfonyl)-benz-(2-nitro-4-acetyl)-anilid werden mit 50,4 g Natriumdithionit in einem Gemisch von 190 ml Äthanol und 200 ml Wasser 2,5 Stunden lang zum Sieden erhitzt. Man dampft zur Trockne ein und verteilt zwischen Methylenchlorid und Wasser. Beim Eindampfen der organischen Phase nach dem Trocknen über Magnesiumsulfat erhält man 2-Methoxy-4-(dimethylamino-formimido-sulfonyl)-benz-(2-amino-4-acetyl)-anilid, welches roh in die nachfolgende Reaktion eingesetzt wird.
Ausbeute: 5,3 g (30 % der Theorie).

d) 0,400 g 2-Methoxy-4-(dimethylamino-formimido-sulfonyl)-benz-(2-amino-4-acetyl)-anilid werden in 10 ml 5n Salzsäure 1 Stunde lang zum Sieden erhitzt. Das auskristallisierende 2-(2'-Methoxy-4'-amidosulfonyl-phenyl)-5-acetyl-benzimidazol wird nach dem Abkühlen abgesaugt, säurefrei gewaschen und getrocknet.
Schmelzpunkt: 269-272°C,
Ausbeute: 280 mg (85 % der Theorie).

Beispiel 35

2-(2'-Methoxy-4'-methylmercapto-phenyl)-5-acetyl-benzimidazol

a) Analog Beispiel 34b) werden 2-Methoxy-4-methylmercapto-benzoylchlorid und 3-Nitro-4-amino-acetophenon zu (2-Methoxy-4-methylmercapto)-benz-(2-nitro-4-acetyl)-anilid umgesetzt und dieses

b) mit Natriumdithionit zu (2-Methoxy-4-methylmercapto)-benz-(2-amino-4-acetyl)-anilid analog Beispiel 34c) reduziert, welches anschließend

c) analog Beispiel 34d) mit siedender Salzsäure zu 2-(2'-Methoxy-4'-methylmercapto-phenyl)-5-acetyl-benzimidazol umgesetzt wird.
Schmelzpunkt: 154-157°C,
Ausbeute: 63 % der Theorie.

## Beispiel 36

2-(2'-Methoxy-4'-methylsulfinyl-phenyl)-5-acetyl-benzimidazol

Hergestellt analog Beispiel 3 aus 2-(2'-Methoxy-4'-methylmercapto-phenyl)-5-acetyl-benzimidazol und Wasserstoffperoxid.
Schmelzpunkt: 184-187°C,
Ausbeute: 59 % der Theorie.

## Beispiel 37

2-(2'-Methoxy-4'-methylsulfonyl-phenyl)-5-acetyl-benzimidazol

Hergestellt analog Beispiel 2 aus 2-(2'-Methoxy-4'-methylmercapto-phenyl)-5-acetyl-benzimidazol und Wasserstoffperoxid.
Schmelzpunkt: 203-205°C,
Ausbeute: 78 % der Theorie.

Beispiel 38

2-(2'-Methoxy-5-methylsulfoximino-phenyl)-1H-imidazo[4,5-b]-pyridin

Hergestellt analog Beispiel 4 aus 2-(2'-Methoxy-5'-methylsulfinyl-phenyl)-1H-imidazo[4,5-b]pyridin und O-Mesitylensulfonyl-acethydroxamsäure-ethylester.
Schmelzpunkt: 263,5°C,
Ausbeute: 83 % der Theorie.

Beispiel 39

2-(2'-Äthoxy-5'-methylsulfoximino-phenyl)-1H-imidazo[4,5-b]-pyridin

Hergestellt analog Beispiel 4 aus 2-(2'-Äthoxy-5'-methylsulfinyl-phenyl)-1H-imidazo[4,5-b]pyridin und O-Mesitylensulfonyl-acethydroxamsäure-ethylester.
Schmelzpunkt: 277°C,
Ausbeute: 62 % der Theorie.

Beispiel 40

2-(2'-Äthoxy-4'-methylsulfoximino-phenyl)-1H-imidazo[4,5-b]-pyridin

Hergestellt analog Beispiel 4 aus 2-(2'-Äthoxy-4'-methylsulfinyl-phenyl)-1H-imidazo[4,5-b]pyridin und O-Mesitylensulfonyl-acethydroxamsäure-ethylester.
Schmelzpunkt: 176-178°C,
Ausbeute: 80 % der Theorie.

**Beispiel 41**

2-(2'-Äthoxy-4'-n-propylsulfoximino-phenyl)-1H-imidazo-[4,5-b]-pyridin

Hergestellt analog Beispiel 4 aus 2-(2'-Äthoxy-4'-n-propylsulfinyl-phenyl)-1H-imidazo[4,5-b]pyridin und O-Mesitylensulfonyl-acethydroxamsäure-ethylester.
Schmelzpunkt: 135-137°C,
Ausbeute: 71 % der Theorie.

**Beispiel 42**

2-(2'-Methoxy-4'-methylsulfoximino-phenyl)-6-methyl-imidazo-[4,5-b]pyridin

Hergestellt analog Beispiel 4 aus 2-(2'-Methoxy-4'-methylsulfinyl-phenyl)-6-methyl-1H-imidazo[4,5-b]pyridin und O-Mesitylensulfonyl-acethydroxamsäure-ethylester.
Schmelzpunkt: 233°C,
Ausbeute: 59 % der Theorie.

**Beispiel 43**

2-[2'-(2-Methylsulfoximino-ethoxy)-4'-methoxy-phenyl]-1H-imidazo[4,5-b]pyridin-mesitylensulfonat

14 g O-Mesitylensulfonyl-acethydroxamsäure-ethylester, gelöst in 20 ml Dioxan werden bei 22-24°C tropfenweise mit 10 ml 90%iger Schwefelsäure unter leichter Außenkühlung versetzt. Man rührt 20 Minuten nach und gießt dann auf 300 ml Eis und schüttelt das freie O-Mesitylensulfonyl-hydroxylamin

mit Methylenchlorid aus und trocknet die Lösung über Natriumsulfat. Zu dieser Lösung werden 6,62 g 2-[2'-(2-Methylsulfinyl-ethoxy)-4'-methoxy-phenyl]-1H-imidazo[4,5-b]pyridin eingetragen. Nach etwa 5 Minuten beginnen sich Kristalle abzuscheiden, man läßt über Nacht rühren. Das Kristallisat wird abgesaugt und aus Äthanol umkristallisiert.
Schmelzpunkt: 163°C,
Ausbeute: 42 % der Theorie.

Beispiel 44

2-(2'-Methoxy-4'-methylsulfoximino-phenyl)-6-chlor-1H-imidazo[4,5-b]pyridin

0,453 g 2-(2'-Methoxy-4'-methylsulfinyl-phenyl)-6-chlor-1H-imidazo[4,5-b]pyridin werden bei 50°C in 9 g Polyphosphorsäure eingerührt und dann portionsweise mit 0,5 g Natriumazid versetzt. Nach Stehen über Nacht verrührt man mit Eis und stellt mit 40%iger Natronlauge auf pH=7,5. Die dabei ausfallenden Kristalle werden abgesaugt und aus Äthanol umkristallisiert.
Schmelzpunkt: 282°C,
Ausbeute: 0,284 g (60 % der Theorie).

Beispiel 45

2-(2'-Methoxy-4'-methylsulfoximino-phenyl)-1H-imidazo[4,5-b]pyridin

Hergestellt analog Beispiel 44 aus 2-(2'-Methoxy-4'-methylsulfinyl-phenyl)-1H-imidazo[4,5-b]pyridin und Natriumazid.
Schmelzpunkt: 236-237°C,
Ausbeute: 86 % der Theorie.

**Beispiel 46**

2-(2'-Methoxy-4'-methylsulfoximino-phenyl)-1H-imidazo[4,5-c] pyridin

Hergestellt analog Beispiel 44 aus 2-(2'-Methoxy-4'-methylsulfinyl-phenyl)-1H-imidazo[4,5-c]pyridin und Natriumazid.
Schmelzpunkt: 253-257°C,
Ausbeute: 62 % der Theorie.

**Beispiel 47**

8-(2'-Methoxy-4'-methylsulfoximino-phenyl)-purin

Hergestellt analog Beispiel 44 aus 8-(2'-Methoxy-4'-methylsulfinyl-phenyl)-purin und Natriumazid.
Schmelzpunkt: 261°C (unter Zersetzung),
Ausbeute: 66 % der Theorie.

**Beispiel 48**

8-(2'-Methoxy-4'-methylsulfoximino-phenyl)-theophyllin-mesitylensulfonat

Hergestellt analog Beispiel 43 aus 8-(2'-Methoxy-4'-methylsulfinyl-phenyl)-theophyllin und O-Mesitylensulfonyl-hydroxylamin.
Schmelzpunkt: 238°C,
Ausbeute: 32 % der Theorie.

Beispiel 49

2-(2'-Methoxy-4'-benzyloxy-phenyl)-5-methylmercapto-benzimidazol

a) Hergestellt analog Beispiel 34b aus 2-Methoxy-4-benzyloxy-benzoylchlorid und 2-Nitro-4-methylmercapto-anilin,
b) das erhaltene 2-Methoxy-4-benzyloxy)benz-(2-nitro-4-methylmercaotp)-anilid wird analog Beispiel 34c mit Natriumdithionit reduziert
c) und das so erhaltene (2-Methoxy-4-benzyloxy)-benz-(2-amino-4-methylmercapto)-anilid wird analog Beispiel 34d mit siedender Salzsäure cyclisiert.
Ausbeute: 81 % der Theorie,
Schmelzpunkt: 238-240°C

Beispiel 50

2-(2'-Methoxy-4'-benzyloxy-phenyl)-5-methylsulfonyl-benzimidazol

Hergestellt analog Beispiel 2 aus 2-(2'-Methoxy-4'-benzyloxy-phenyl)-5-methylmercapto-benzimidazol und Wasserstoffperoxid.
Ausbeute: 92 % der Theorie
Schmelzpunkt: 156-159°C.

Beispiel 51

2-(2'-Methoxy-4'-hydroxy-phenyl)-5-methylsulfonyl-benzimidazol

Hergestellt aus 2-(2'-Methoxy-5-benzyloxy-phenyl)-5-methyl-

sulfonyl-benzimidazol durch katalytische Hydrierung mit Wasserstoff bei Raumtemperatur in Dimethylformamid.
Ausbeute: 60 % der Theorie,
Schmelzpunkt: 255-260°C

**Beispiel 52**

**2-(2'-Methoxy-4'-methansulfonyloxy-phenyl)-5-methylsulfonyl-benzimidazol**

Hergestellt aus 2-(2'-Methoxy-4'-hydroxy-phenyl)-5-methylsulfonyl-benzimidazol und Methansulfonylchlorid in Pyridin.
Ausbeute: 68 % der Theorie,
Schmelzpunkt: 125-129°C.

**Beispiel 53**

**5-Cyano-2-(2'-methoxy-4'-methansulfonylamino-phenyl)-benzimidazol**

Hergestellt aus 2-Methoxy-4-methansulfonylamino-benzoesäure und 3,4-Diamino-benzonitril analog Beispiel 7.
Ausbeute: 17,5 % der Theorie,
Schmelzpunkt: 293-295°C

| | | | | | | | | |
|---|---|---|---|---|---|---|---|---|
| Ber.: | C | 56,13 | H | 4,12 | N | 16,36 | S | 9,37 |
| Gef.: | | 56,34 | | 4,35 | | 16,28 | | 9,44 |

0200707

Beispiel 54

2-(2'-Methoxy-4'-methansulfonylamino-phenyl)-imidazo-[4,5-b]naphthalin-hydrat

Hergestellt aus 2-Methoxy-4-methansulfonylamino-benzoesäure und 2,3-Diamino-naphthalin analog Beispiel 7.
Ausbeute: 42 % der Theorie,
Schmelzpunkt: amorph, ab ca. 100°C

| | | | | | | |
|---|---|---|---|---|---|---|
| Ber.: | C | 59,20 | H | 4,97 | N | 10,90 |
| Gef.: | | 59,71 | | 5,04 | | 11,13 |

Beispiel 55

5-Aminosulfonyl-2-(2'-methoxy-4'-methylmercapto-phenyl)-benzimidazol

Hergestellt aus 2-Methoxy-4-methylmercapto-benzoesäure und 4-Aminosulfonyl-1,2-diamino-benzol analog Beispiel 7.
Ausbeute: 18,9 % der Theorie,
Schmelzpunkt: 274-276°C

| | | | | | | | | |
|---|---|---|---|---|---|---|---|---|
| Ber.: | C | 51,56 | H | 4,33 | N | 12,03 | S | 18,35 |
| Gef.: | | 51,30 | | 4,48 | | 11,80 | | 18,51 |

Beispiel 56

5-Acetyl-2-(2'-methoxy-4'-methansulfonylamino-phenyl)-benzimidazol

Hergestellt aus 2-Methoxy-4-methansulfonylamino-benzoesäure und 3,4-Diamino-acetophenon analog Beispiel 7.

Ausbeute: 79 % der Theorie,
Schmelzpunkt: 141-143°C

| | | | | | | | | |
|---|---|---|---|---|---|---|---|---|
| Ber.: | C | 56,81 | H | 4,77 | N | 11,69 | S | 8,92 |
| Gef.: | | 55,20 | | 5,09 | | 11,52 | | 8,79 |

## Beispiel 57

5-Benzoyl-2-(2'-methoxy-4'-methansulfonylamino-phenyl)-benzimidazol

Hergestellt aus 2-Methoxy-4-methansulfonylamino-benzoesäure und 3,4-Diamino-benzophenon analog Beispiel 7.
Ausbeute: 62 % der Theorie,
Schmelzpunkt: 139-141°C

| | | | | | | | | |
|---|---|---|---|---|---|---|---|---|
| Ber.: | C | 62,69 | H | 4,54 | N | 9,97 | S | 7,61 |
| Gef.: | | 62,44 | | 4,77 | | 9,87 | | 7,63 |

## Beispiel 58

2-Amino-8-(2'-methoxy-4'-methylmercapto-phenyl)-purin

Hergestellt aus 2-Methoxy-4-methylmercapto-benzoesäure und 2,4,5-Triamino-pyrimidin analog Beispiel 7.
Ausbeute: 17 % der Theorie,
Schmelzpunkt: 212-214°C

| | | | | | | | | |
|---|---|---|---|---|---|---|---|---|
| Ber.: | C | 54,34 | H | 4,56 | N | 24,37 | S | 11,16 |
| Gef.: | | 54,63 | | 4,66 | | 24,36 | | 10,76 |

Beispiel 59

6-Cyano-2-(2'-methoxy-4'-methylmercapto-phenyl)-imidazo-[4,5-b]pyridin

Hergestellt aus 2-Methoxy-4-methylmercapto-benzoesäure und 5-Cyano-2,3-diamino-pyridin analog Beispiel 7.
Ausbeute: 28 % der Theorie,
Schmelzpunkt: 268-270°C

| | | | | | | | | |
|---|---|---|---|---|---|---|---|---|
| Ber.: | C | 60,78 | H | 4,08 | N | 18,90 | S | 10,82 |
| Gef.: | | 60,60 | | 4,25 | | 19,10 | | 11,01 |

Beispiel 60

5-Aminosulfonyl-2-(2'-methoxy-4'-methylsulfonyl-phenyl)-benzimidazol

Hergestellt aus 5-Aminosulfonyl-2-(2'-methoxy-4'-methylmercapto-phenyl)-benzimidazol und Wasserstoffperoxid analog Beispiel 2.
Ausbeute: 38,3 % der Theorie,
Schmelzpunkt: 298-300°C

| | | | | | | | | |
|---|---|---|---|---|---|---|---|---|
| Ber.: | C | 47,24 | H | 3,96 | N | 11,02 | S | 16,81 |
| Gef.: | | 46,92 | | 4,58 | | 10,82 | | 16,50 |

Beispiel 61

2-Amino-8-(2'-methoxy-4'-methylsulfinyl-phenyl)-purin

Hergestellt aus 2-Amino-8-(2'-methoxy-4'-methylmercapto-phenyl)-purin und Wasserstoffperoxid analog Beispiel 3.

0209707

Ausbeute: 53 % der Theorie,
Schmelzpunkt: amorph, ab ca. 150°C.

| | | | | | | | | |
|---|---|---|---|---|---|---|---|---|
| Ber.: | C | 51,47 | H | 4,32 | N | 23,08 | S | 10,57 |
| Gef.: | | 51,40 | | 4,28 | | 22,28 | | 10,24 |

## Beispiel 62

2-(2'-Methoxy-4'-methoxycarbonylamino-phenyl)-imidazo[4,5-c]-pyridin

a) 3,27 g (30 mMol) 3,4-Diamino-pyridin und 5,02 g (30 mMol) 4-Amino-2-methoxy-benzoesäure werden zwei Stunden lang unter heftigem Rühren in 50 g Polyphosphorsäure auf 130°C erhitzt. Nach dem Abkühlen wird mit ca. 100 ml Wasser versetzt, der ausgefallene gelbe Niederschlag abgesaugt, in 50 ml Wasser suspendiert und mit konzentriertem Ammoniak schwach alkalisch gestellt. Das ungelöste Produkt wird abgesaugt und im Umlufttrockenschrank bei 50°C getrocknet. Man erhält 6,8 g 2-(4'-Amino-2'-methoxy-phenyl)-imidazo[4,5-c]pyridin, das nicht weiter gereinigt, sondern gleich weiter umgesetzt wird.

b) 1,2 g (5 mMol) 2-(4'-Amino-2'-methoxy-phenyl)-imidazo-[4,5-c]pyridin, gelöst in 20 ml Pyridin, werden bei -10°C mit 0,7 ml Chlorameisensäuremethylester versetzt. Danach wird langsam auf Raumtemperatur erwärmt und weitere zwei Stunden lang gerührt. Das Reaktionsgemisch wird dann zur Trockne eingedampft, der Rückstand eine Stunde lang in 30 ml 5%iger Natriumhydrogencarbonat-Lösung gerührt, abgesaugt, getrocknet und durch Säulenchromatographie über Kieselgel gereinigt (Elutionsmittel: Methylenchlorid mit 1-12 % Ethanol).
Ausbeute: 67 % der Theorie,
Schmelzpunkt: 229-230°C

| | | | | | | |
|---|---|---|---|---|---|---|
| Ber.: | C | 60,40 | H | 4,73 | N | 18,78 |
| Gef.: | | 60,50 | | 5,02 | | 18,45 |

Beispiel 63

5-Ethylaminocarbonylamino-2-(2'-methoxy-4'-methansulfonyloxy-phenyl)-benzimidazol

1,0 g (3 mMol) 5-Amino-2-(4'-methansulfonyloxy-2'-methoxy-phenyl)-benzimidazol wird in 30 ml Tetrahydrofuran gelöst, 2,5 ml Ethylisocyanat hinzugegeben und zum Rückfluß erhitzt. Nach 15 Minuten dampft man das Lösungsmittel und überschüssiges Ethylisocyanat im Vakuum ab. Der erhaltene Rückstand wird über 250 g Aluminiumoxid (neutral) chromatographiert (Elutionsmittel: Methylenchlorid mit 4 % Ethanol).
Ausbeute: 27,5 % der Theorie,
Schmelzpunkt: 194-196°C

| | | | | |
|---|---|---|---|---|
| Ber.: | C 53,45 | H 4,98 | N 13,85 | S 7,93 |
| Gef.: | 53,02 | 5,03 | 14,09 | 7,77 |

Beispiel 64

5-Isopropylaminocarbonylamino-2-(2'-methoxy-4'-methansulfonyloxy-phenyl)-benzimidazol

Hergestellt aus 5-Amino-2-(2'-methoxy-4'-methansulfonyloxy-phenyl)-benzimidazol und Isopropylisocyanat analog Beispiel 63.
Ausbeute: 27,1 % der Theorie,
Schmelzpunkt: 189-190°C

| | | | | |
|---|---|---|---|---|
| Ber.: | C 54,53 | H 5,30 | N 13,39 | S 7,60 |
| Gef.: | 54,23 | 5,12 | 13,14 | 8,71 |

0209707

**Beispiel 65**

**8-(2'-Methoxy-4'-methylaminocarbonylamino-phenyl)-purin**

Hergestellt aus 8-(4'-Amino-2'-methoxy-phenyl)-purin und Methylisocyanat analog Beispiel 63.
Ausbeute: 17,3 % der Theorie,
Schmelzpunkt: 269-271°C

| | | | | | | |
|---|---|---|---|---|---|---|
| Ber.: | C | 53,17 | H | 5,10 | N | 26,57 |
| Gef.: | | 53,79 | | 4,99 | | 26,07 |

**Beispiel 66**

**4-Cyano-2-(4'-methansulfonyloxy-2'-methoxy-phenyl)-benzimidazol**

3,0 g (11,3 mMol) 4-Cyano-2-(4'-hydroxy-2'-methoxy-phenyl)-benzimidazol, gelöst in 90 ml 1n Natronlauge, wird unter Rühren tropfenweise mit 5 ml Methansulfonylchlorid versetzt. Durch allmähliches Zugeben von weiterer 1n Natronlauge wird dabei der pH-Wert der Reaktionslösung bei 10,0 bis 10,5 gehalten. Das ausgefallene Produkt wird anschließend abgesaugt, mit Wasser gewaschen, getrocknet und durch Säulenchromatographie über 300 g Aluminiumoxid (neutral, Aktivitätsstufe II) gereinigt (Elutionsmittel: Methylenchlorid).
Ausbeute: 72 % der Theorie,
Schmelzpunkt: 225-227°C

| | | | | | | | | |
|---|---|---|---|---|---|---|---|---|
| Ber.: | C | 55,97 | H | 3,82 | N | 12,24 | S | 9,34 |
| Gef.: | | 55,90 | | 3,71 | | 12,21 | | 9,30 |

Beispiel 67

4-Aminocarbonyl-2-(4'-methansulfonyloxy-2'-methoxy-phenyl)-benzimidazol

1,0 g (2,9 mMol) 4-Cyano-2-(4'-methansulfonyloxy-2'-methoxy-phenyl)-benzimidazol wird in 20 ml konzentrierter Schwefelsäure 18 Stunden lang bei Raumtemperatur gerührt, die Lösung anschließend auf 50 g Eis gegossen und unter Eiskühlung mit konzentriertem Ammoniak neutralisiert. Das ausgefallene Produkt wird abgesaugt, getrocknet und durch Säulenchromatographie über Kieselgel gereinigt (Elutionsmittel: Methylenchlorid mit 5 % Ethanol).

Ausbeute: 79 % der Theorie,

Schmelzpunkt: ab 250°C (sintert)

| | | | | | | | | |
|---|---|---|---|---|---|---|---|---|
| Ber.: | C | 53,18 | H | 4,18 | N | 11,63 | S | 8,87 |
| Gef.: | | 53,05 | | 4,13 | | 11,66 | | 9,07 |

Beispiel 68

5-Methylaminocarbonylmethylamino-2-(4'-methansulfonyloxy-2'-methoxy-phenyl)-benzimidazol

Hergestellt aus 5-Methylaminocarbonylmethylamino-2-(4'-hydroxy-2'-methoxy-phenyl)-benzimidazol und Methansulfonylchlorid analog Beispiel 66.

Ausbeute: 63 % der Theorie,

Schmelzpunkt: 210-212°C

| | | | | | | | | |
|---|---|---|---|---|---|---|---|---|
| Ber.: | C | 53,45 | H | 4,98 | N | 13,85 | S | 7,93 |
| Gef.: | | 53,36 | | 5,36 | | 13,70 | | 8,06 |

**Beispiel 69**

**4-Methyl-2-(4'-methansulfonyloxy-2'-methoxy-phenyl)-benzimidazol**

Hergestellt aus 4-Methyl-2-(4'-hydroxy-2'-methoxy-phenyl)-benzimidazol und Methansulfonylchlorid analog Beispiel 66.
Ausbeute: 60 % der Theorie,
Schmelzpunkt: 195-196°C

| | | | | | | | | |
|---|---|---|---|---|---|---|---|---|
| Ber.: | C | 57,82 | H | 4,85 | N | 8,43 | S | 9,65 |
| Gef.: | | 57,54 | | 4,73 | | 8,40 | | 9,50 |

**Beispiel 70**

**2-Amino-8-(4'-methansulfonyloxy-2'-methoxy-phenyl)-purin**

Hergestellt aus 2-Amino-8-(4'-methansulfonyloxy-2'-methoxy-phenyl)-purin und Methansulfonylchlorid analog Beispiel 66.
Ausbeute: 41 % der Theorie,
Schmelzpunkt: 246-247°C

| | | | | | | | | |
|---|---|---|---|---|---|---|---|---|
| Ber.: | C | 46,56 | H | 3,91 | N | 20,88 | S | 9,56 |
| Gef.: | | 46,63 | | 4,12 | | 20,74 | | 9,43 |

**Beispiel 71**

**4-Methoxycarbonyl-2-(4'-methansulfonyloxy-2'-methoxy-phenyl)-benzimidazol**

Eine Lösung aus 400 mg (1,34 mMol) 4-Methoxycarbonyl-2-(4'-hydroxy-2'-methoxy-phenyl)-benzimidazol, 10 ml Pyridin und 0,8 ml Methansulfonylchlorid wird 18 Stunden lang bei Raumtemperatur gerührt, dann unter Vakuum eingeengt, der Rückstand mit 15 ml Wasser verrührt und mit 0,5n Salzsäure auf pH 8 eingestellt. Die so erhaltene Lösung wird dreimal mit je 10 ml Methylenchlorid extrahiert, die Extrakte getrocknet und eingeengt. Der feste Rückstand wird durch Säulenchromatographie über 150 g Kieselgel gereinigt (Elutionsmittel: Methylenchlorid mit 1 % Ethanol).

Ausbeute: 79,5 % der Theorie,

Schmelzpunkt: 182-183°C

| | | | | | | |
|---|---|---|---|---|---|---|
| Ber.: | C | 54,25 | H | 4,28 | N | 7,44 |
| Gef.: | | 53,97 | | 4,08 | | 7,31 |

Beispiel 72

2-(4'-Methansulfonyloxy-2'-methoxy-phenyl)-imidazo[4,5-b]-naphthalin

Hergestellt aus 2-(4'-Hydroxy-2'-methoxy-phenyl)-imidazo-[4,5-b]naphthalin und Methansulfonylchlorid analog Beispiel 71.

Ausbeute: 9 % der Theorie,

Schmelzpunkt: 195-197°C

| | | | | | | |
|---|---|---|---|---|---|---|
| Ber.: | C | 61,94 | H | 4,38 | N | 7,60 |
| Gef.: | | 61,90 | | 4,34 | | 7,66 |

Beispiel 73

5-Acetaminomethyl-2-(2'-methoxy-4'-methansulfonyloxy-phenyl)-benzimidazol

Hergestellt aus 5-Acetaminomethyl-2-(4'-hydroxy-2'-methoxy-phenyl)-benzimidazol und Methansulfonylchlorid analog Beispiel 71.

0209707

Ausbeute: 17,3 % der Theorie,
Schmelzpunkt: amorph

| | | | | | | | | |
|---|---|---|---|---|---|---|---|---|
| Ber.: | C | 55,52 | H | 4,92 | N | 10,79 | S | 8,23 |
| Gef.: | | 55,63 | | 5,05 | | 10,45 | | 8,05 |

## Beispiel 74

**5-Acetyl-2-(4'-methansulfonyloxy-2'-methoxy-phenyl)-benzimidazol**

Hergestellt aus 5-Acetyl-2-(4'-hydroxy-2'-methoxy-phenyl)-benzimidazol und Methansulfonylchlorid analog Beispiel 71.
Ausbeute: 44 % der Theorie,
Schmelzpunkt: 182-184°C

| | | | | | | | | |
|---|---|---|---|---|---|---|---|---|
| Ber.: | C | 56,66 | H | 4,48 | N | 7,77 | S | 8,90 |
| Gef.: | | 56,62 | | 5,04 | | 7,76 | | 9,87 |

## Beispiel 75

**5-(Phenyl-methoxymethyl)-2-(4'-methansulfonyloxy-2'-methoxy-phenyl)-benzimidazol**

Hergestellt aus 5-(Phenyl-methoxymethyl)-2-(4'-hydroxy-2'-methoxy-phenyl)-benzimidazol und Methansulfonylchlorid analog Beispiel 71.
Ausbeute: 8 % der Theorie,
Schmelzpunkt: amorph

| | | | | | | | | |
|---|---|---|---|---|---|---|---|---|
| Ber.: | C | 62,99 | H | 5,06 | N | 6,39 | S | 7,31 |
| Gef.: | | 62,75 | | 4,93 | | 6,45 | | 7,12 |

Beispiel A

Tabletten zu 100 mg 5-Cyano-2-(4'-methansulfonylamino-2'-methoxy-phenyl)-benzimidazol

Zusammensetzung

1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm

Trocknen: Umlufttrockenschrank 50°C

Trockensiebung: 1 mm

Dem Granulat die restlichen Hilfsstoffe zumischen und Endmischung zu Tabletten verpressen.

Tablettengewicht: 175 mg

Stempel: 8 mm

Beispiel B

Dragées zu 50 mg 5-Cyano-2-(4'-methansulfonylamino-2'-methoxy-phenyl)-benzimidazol

Zusammensetzung:

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

Feuchtsiebung: 1,0 mm

Trockensiebung: 1,0 mm,

Trocknung: 50°C im Umlufttrockenschrank

Granulat und restliche Hilfsstoffe mischen und zu Kernen verpressen.

Kerngewicht: 80 mg

Stempel: 6 mm

Wölbungsradius: 5 mm

Die fertigen Kerne werden auf übliche Weise mit einem Zuckerüberzug im Dragierkessel versehen.

Dragéegewicht: 120 mg

## Beispiel C

**Suppositorien zu 75 mg 5-Cyano-2-(4'-methansulfonylamino-2-phenyl)-benzimidazol**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

**Herstellungsverfahren:**

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

## Beispiel D

Ampullen zu 50 mg 5-Cyano-2-(4'-methansulfonylamino-2'-methoxy-phenyl)-benzimidazol

1 Ampulle enthält:

| | |
|---|---|
| Wirksubstanz | 50,0 mg |
| Ethoxylierte Hydroxystearinsäure | 750,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

## Beispiel E

Tropfen zu 100 mg 5-Cyano-2-(4'-methansulfonylamino-2'-methoxy-phenyl)-benzimidazol

| | | |
|---|---|---|
| Wirksubstanz | | 1,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Ethanol | | 40,0 g |
| Dest. Wasser | ad | 100,0 ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. Neue 2-Arylimidazole der allgemeinen Formel

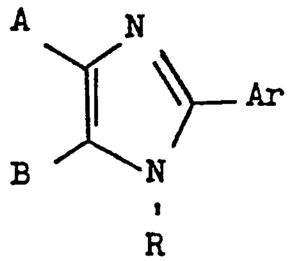 (I)

in der

R ein Wasserstoffatom oder eine Alkylgruppe,

Ar eine Gruppe der Formel

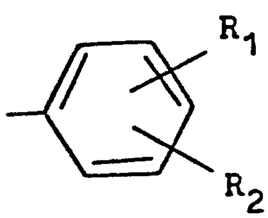 oder 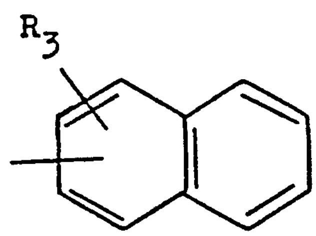 und

A und B zusammen mit den beiden dazwischen liegenden Kohlenstoffatomen eine Gruppe der Formel

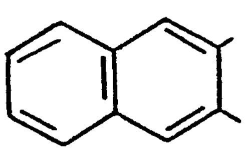, 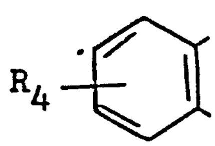, 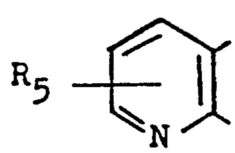, 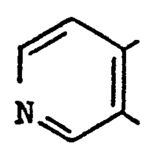,

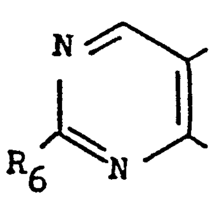 oder 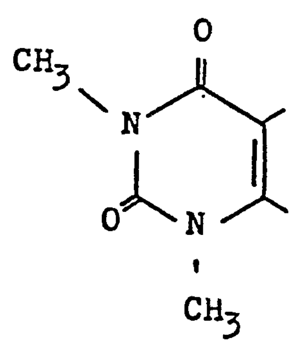 darstellen,

wobei

$R_1$ eine Alkylsulfenyl-, Alkylsulfinyl-, Alkylsulfonyl- oder Alkylsulfoximinogruppe, eine am Stickstoffatom durch eine Alkanoyl-, Alkylsulfonyl- oder Hydroxycarbonyl-alkylen-carbonylgruppe substituierte Alkylsulfoximinogruppe, eine Ethoxy- oder n-Propoxygruppe, die jeweils endständig durch eine Alkylsulfenyl-, Alkylsulfinyl-, Alkylsulfonyl- oder Alkylsulfoximinogruppe substituiert ist, eine Alkoxycarbonylamino- oder N-Alkylaminocarbonyl-aminogruppe oder auch in 4-Stellung eine Hydroxy-, Phenylalkoxy-, Aminosulfonyl-, Alkylsulfonyloxy- oder Alkylsulfonylaminogruppe, wenn

a) A und B zusammen mit den beiden dazwischen liegenden Kohlenstoffatomen eine

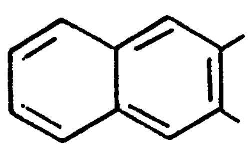

-Gruppe oder

b) $R_4$ eine Alkyl-, Alkanoyl-, N-Alkanoyl-aminomethyl-, Benzoyl-, Phenyl-methoxymethyl-, Aminosulfonyl-, N-Ethylaminocarbonyl-amino-, N-n-Propylaminocarbonyl-amino-, N-Isopropylaminocarbonyl-amino- oder N-Alkyl-N-alkylaminocarbonyl-aminogruppe oder

c) $R_6$ die Aminogruppe darstellt, wobei mindestens einer der Reste die unter a), b) und c) aufgeführten Bedeutungen darstellen muß,

$R_2$ ein Wasserstoffatom oder eine Alkoxygruppe,

$R_3$ eine Ethoxy- oder n-Propoxygruppe, die jeweils endständig durch eine Alkylsulfenyl-, Alkylsulfinyl-, Alkylsulfonyl- oder Alkylsulfoximinogruppe substituiert ist,

$R_4$ eine Alkyl-, Alkoxycarbonyl-, Alkanoyl-, N-Alkanoylaminomethyl-, Benzoyl-, Phenyl-methoxymethyl-, Aminocarbon-

yl-, Cyano-, Trifluormethyl-, Aminosulfonyl-, N-Alkylaminocarbonyl-amino- oder N-Alkyl-N-alkylaminocarbonyl-amino-gruppe,

$R_5$ ein Wasserstoff- oder Halogenatom, eine Alkyl- oder Cyanogruppe und

$R_6$ ein Wasserstoffatom oder eine Aminogruppe unter der Voraussetzung bedeuten, daß

a) $R_1$ keine Alkylmercaptogruppe in 4-Stellung darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen,

b) $R_1$ keine Alkylsulfinylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

c) $R_1$ keine Alkylsulfonylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

d) $R_1$ keine Alkylsulfoximinogruppe in 4-Stellung darstellt, wenn $R_4$ in 5-Stellung eine Cyano- oder Carbamidogruppe und gleichzeitig $R_2$ in 2-Stellung eine Methoxy- oder Ethoxygruppe und R ein Wasserstoffatom darstellen, oder

e) $R_1$ in 4-Stellung keine Hydroxy- oder Phenylalkoxygruppe darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen,

wobei die vorstehend erwähnten Alkyl-, Alkylen-, Alkoxy- und Alkanoylteile jeweils 1 bis 3 Kohlenstoffatome enthalten können, bedeuten,

sowie die Verbindungen

5-Cyano-2-(4'-methansulfonylamino-2'-methoxy-phenyl)-benzimidazol,

4-Methyl-2-(4'-methansulfonyloxy-2'-methoxy-phenyl)-benzimidazol,

4-Cyano-2-(4'-methansulfonyloxy-2'-methoxy-phenyl)-benzimidazol,

4-Aminocarbonyl-2-(4'-methansulfonyloxy-2'-methoxy-phenyl)-benzimidazol und

4-Methoxycarbonyl-2-(4'-methansulfonyloxy-2'-methoxy-phenyl)-benzimidazol,

deren Tautomere, wenn R ein Wasserstoffatom darstellt, und deren Säureadditionssalze.

2. Neue 2-Arylimidazole der allgemeinen Formel I gemäß Anspruch 1, in der

R ein Wasserstoffatom oder die Methylgruppe,

$R_1$ eine Methylsulfenyl-, Ethylsulfenyl-, Methylsulfinyl-, Ethylsulfinyl-, Methylsulfonyl-, Ethylsulfonyl-, Methylsulfoximino-, Ethylsulfoximino- oder n-Propylsulfoximinogruppe, eine am Stickstoffatom durch eine Acetyl-, Methansulfonyl- oder Hydroxycarbonyl-äthylencarbonylgruppe substituierte Methylsulfoximinogruppe, eine Ethoxygruppe, die endständig

durch eine Methylsulfoximinogruppe substituiert ist, eine Methoxycarbonylamino- oder N-Methylaminocarbonyl-aminogruppe oder auch in 4-Stellung eine Hydroxy-, Benzyloxy-, Aminosulfonyl-, Methansulfonyloxy- oder Methansulfonylaminogruppe, wenn

a) A und B zusammen mit den beiden dazwischen liegenden Kohlenstoffatomen eine

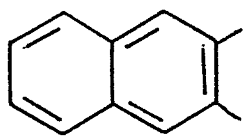

-Gruppe oder

b) $R_4$ eine Methyl-, Acetyl-, N-Acetyl-aminomethyl-, Benzoyl-, Phenyl-methoxymethyl-, Aminosulfonyl-, N-Ethylaminocarbonyl-amino-, N-Isopropylaminocarbonyl-amino- oder N-Methyl-N-methylaminocarbonyl-aminogruppe oder

c) $R_6$ die Aminogruppe darstellt, wobei mindestens einer der Reste die unter a), b) und c) aufgeführten Bedeutungen darstellen muß,

$R_2$ ein Wasserstoffatom, eine Methoxy- oder eine Ethoxygruppe,

$R_3$ eine Ethoxygruppe, die endständig durch eine Methylsulfenyl-, Methylsulfinyl-, Methylsulfonyl- oder Methylsulfoximinogruppe substituiert ist,

$R_4$ eine Methyl-, Methoxycarbonyl-, Acetyl-, N-Acetylaminomethyl-, Benzoyl-, Phenyl-methoxymethyl-, Aminocarbonyl-, Cyano-, Trifluoromethyl-, Aminosulfonyl-, N-Ethylaminocarbonyl-amino-, N-Isopropylaminocarbonyl-amino- oder N-Methyl-N-methylaminocarbonyl-aminogruppe,

$R_5$ ein Wasserstoff- oder Chloratom, eine Methyl- oder Cyanogruppe und

$R_6$ ein Wasserstoffatom oder eine Aminogruppe unter der Voraussetzung bedeuten, daß

a) $R_1$ keine Methyl- oder Ethylmercaptogruppe in 4-Stellung darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen,

b) $R_1$ keine Methyl- oder Ethylsulfinylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

c) $R_1$ keine Methyl- oder Ethylsulfonylgruppe in 4-Stellung darstellt, wenn $R_6$ ein Wasserstoffatom oder $R_4$ in 5-Stellung eine Cyano- oder Aminocarbonylgruppe und gleichzeitig $R_2$ in 2-Stellung eine Methoxy- oder Ethoxygruppe und R ein Wasserstoffatom darstellen,

d) $R_1$ keine Methyl- oder Ethylsulfoximinogruppe in 4-Stellung darstellt, wenn $R_4$ in 5-Stellung eine Cyano- oder Carbamidogruppe und gleichzeitig $R_2$ in 2-Stellung eine Methoxy- oder Ethoxygruppe und R ein Wasserstoffatom darstellen, oder

e) $R_1$ in 4-Stellung keine Hydroxy- oder Benzyloxygruppe darstellt, wenn $R_6$ ein Wasserstoffatom oder $R_4$ in 5-Stellung eine Methoxycarbonyl-, Aminocarbonyl- oder Cyanogruppe und gleichzeitig $R_2$ in 2-Stellung eine Methoxy- oder Ethoxygruppe und R ein Wasserstoffatom darstellen, bedeuten, sowie die Verbindungen

5-Cyano-2-(4'-methansulfonylamino-2'-methoxy-phenyl)-benzimidazol,

4-Methyl-2-(4'-methansulfonyloxy-2'-methoxy-phenyl)-benzimidazol,

4-Cyano-2-(4'-methansulfonyloxy-2'-methoxy-phenyl)-benzimidazol,

4-Aminocarbonyl-2-(4'-methansulfonyloxy-2'-methoxy-phenyl)-benzimidazol und

4-Methoxycarbonyl-2-(4'-methansulfonyloxy-2'-methoxy-phenyl)-benzimidazol,

deren Tautomere, wenn R ein Wasserstoffatom darstellt, und deren Säureadditionssalze.

3. 2-[2'-Methoxy-4'-(N-acetyl-methylsulfoximino-phenyl)]-5-cyano-benzimidazol,

2-(2'-Methoxy-4'-methylsulfinyl-phenyl)-5-acetyl-benzimidazol,

2-(2'-Methoxy-4'-methylsulfonyl-phenyl)-5-acetyl-benzimidazol,

5-Cyano-2-(4'-methansulfonylamino-2'-methoxyphenyl)-benzimidazol,

2-(2'-Methoxy-4'-methoxycarbonylamino-phenyl)-imidazo[4,5-c]-pyridin,

6-Cyano-2-(2'-methoxy-4'-methylmercapto-phenyl)-imidazo-[4,5-b]pyridin,

2-Amino-8-(2'-methoxy-4'-methylsulfinyl-phenyl)-purin,

2-(2'-Methoxy-4'-methylsulfoximino-phenyl)-1H-imidazo[4,5-c]-pyridin,

5-Aminocarbonyl-2-(4'-methansulfonyloxy-2'-methoxy-phenyl)-benzimidazol und

5-Acetyl-2-(4'-methansulfonyloxy-2'-methoxy-phenyl)-benzimidazol,

deren Tautomere und deren Säureadditionssalze.

4. 5-Cyano-2-(4'-methansulfonylamino-2'-methoxyphenyl)-benzimidazol, dessen Tautomere und dessen Säureadditionssalze.

5. 2-(2'-Methoxy-4'-methylsulfonyl-phenyl)-5-acetyl-benzimidazol, dessen Tautomere und dessen Säureadditionssalze.

6. Physiologisch verträgliche Säureadditionssalze der Verbindungen gemäß den Ansprüchen 1 bis 5.

7. Arzneimittel, enthaltend eine Verbindung gemäß den Ansprüchen 1 bis 5 oder ein physiologisch verträgliches Säureadditionssalz gemäß Anspruch 6 neben gegebenenfalls einem oder mehreren inerten Trägerstoffen und/oder Verdünnungsmitteln.

8. Verwendung einer Verbindung gemäß den Ansprüchen 1 bis 5 oder eines physiologischen verträglichen Säureadditionssalzes gemäß Anspruch 6 zur Herstellung eines Arzneimittels, welches zur Behandlung von Herzinsuffizienzen unterschiedlicher Genese, zur Behandlung und zur Prophylaxe thrombo-embolischer Erkrankungen, zur Prophylaxe der Arteriosklerose und zur Metastasenprophylaxe geeignet ist.

9. Verfahren zur Herstellung eines Arzneimittels gemäß Anspruch 7, dadurch gekennzeichnet, daß auf nichtchemischem Wege eine Verbindung gemäß den Ansprüchen 1 bis 5 oder ein physiologisch verträgliches Säureadditionssalz gemäß Anspruch 6 in einen oder mehrere inerte Trägerstoffe und/oder Verdünnungsmittel eingearbeitet wird.

10. Verfahren zur Herstellung der Verbindungen gemäß den Ansprüchen 1 bis 6, dadurch gekennzeichnet, daß

a) eine gegebenenfalls im Reaktionsgemisch hergestellte Verbindung der allgemeinen Formel

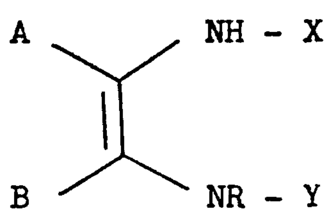

(II)

in der
A, B und R wie in den Ansprüchen 1 bis 5 definiert sind, einer der Reste X oder Y ein Wasserstoffatom und der andere der beiden Reste X und Y oder beide Reste X und Y eine Gruppe der Formel

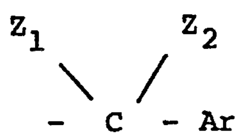

darstellen, in der

Ar wie in den Ansprüchen 1 bis 5 definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der $R_1$ eine Alkylsulfinyl-, Alkylsulfonyl- oder Alkylsulfoximinogruppe, eine am Stickstoffatom durch eine Alkanoyl-, Alkylsulfonyl- oder Hydroxycarbonyl-alkylencarbonylgruppe substituierte Alkylsulfoximinogruppe, eine Ethoxy- oder n-Propoxygruppe, die endständig durch eine Alkylsulfinyl-, Alkylsulfonyl- oder Alkylsulfoximinogruppe substituiert ist, oder $R_3$ eine Ethoxy- oder n-Propoxygruppe, die endständig durch eine Alkylsulfinyl-, Alkylsulfonyl- oder Alkylsulfoximinogruppe substituiert ist, darstellen, eine Verbindung der allgemeinen Formel

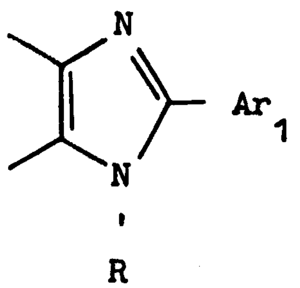

,(III)

in der

A, B und R wie in den Ansprüchen 1 bis 5 definiert sind und $Ar_1$ die für Ar in den Ansprüchen 1 bis 5 erwähnten Bedeutungen besitzt, wobei jedoch $R_1$ eine Alkylsulfenyl-, Alkylsulfinyl- oder Alkylsulfiminogruppe, eine am Stickstoffatom durch eine Alkanoyl-, Alkylsulfonyl- oder Hydroxycarbonyl-alkylencarbonylgruppe substituierte Alkylsulfiminogruppe, eine Ethoxy- oder n-Propoxygruppe, die endständig durch eine Alkylsulfenyl-, Alkylsulfinyl- oder Alkylsulfiminogruppe substituiert ist, oder $R_3$ eine Ethoxy- oder n-Propoxygruppe, die endständig durch eine Alkylsulfenyl-, Alkylsulfinyl- oder Alkylsulfiminogruppe substituiert ist, darstellen muß, oxidiert wird oder

c) zur Herstellung von Verbindungen der allgemeinen Formel I, in der $R_1$ eine Alkylsulfoximinogruppe, eine Ethoxy- oder n-Propoxygruppe, die endständig durch eine Alkylsulfoximino-

gruppe substituiert ist, oder $R_3$ eine Ethoxy- oder n-Propoxygruppe, die endständig durch eine Alkylsulfoximinogruppe substituiert ist, darstellt, ein Sulfoxid der allgemeinen Formel

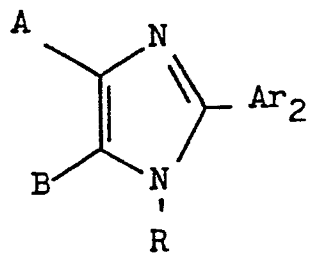

,(IV)

in der
A, B und R wie in den Ansprüchen 1 bis 5 definiert sind und $Ar_2$ die für Ar in den Ansprüchen 1 bis 5 erwähnten Bedeutungen besitzt, wobei jedoch $R_1$ eine Alkylsulfinylgruppe, eine Ethoxy- oder n-Propoxygruppe, die endständig durch eine Alkylsulfinylgruppe substituiert ist, oder $R_3$ eine Ethoxy- oder n-Propoxygruppe, die endständig durch eine Alkylsulfinylgruppe substituiert ist, darstellen muß, mit gegebenenfalls im Reaktionsgemisch gebildeter Stickstoffwasserstoffsäure umgesetzt wird oder

d) zur Herstellung von Verbindungen der allgemeinen Formel I, in der $R_1$ eine Alkylsulfoximinogruppe, eine Ethoxy- oder n-Propoxygruppe, die endständig durch eine Alkylsulfoximinogruppe substituiert ist, oder $R_3$ eine Ethoxy- oder n-Propoxygruppe, die endständig durch eine Alkylsulfoximinogruppe substituiert ist, darstellt, ein Sulfoxid der allgemeinen Formel

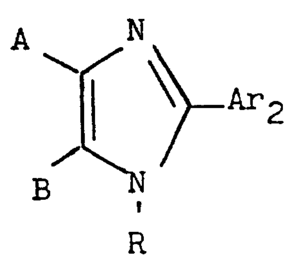

,(IV)

in der
A, B und R wie in den Ansprüchen 1 bis 5 definiert sind und $Ar_2$ die für Ar in den Ansprüchen 1 bis 5 erwähnten Bedeutungen besitzt, wobei jedoch $R_1$ eine Alkylsulfinylgruppe, eine Ethoxy- oder n-Propoxygruppe, die endständig durch eine Alkylsulfinylgruppe substituiert ist, oder $R_3$ eine Ethoxy- oder n-Propoxygruppe, die endständig durch eine Alkylsulfinylgruppe substituiert ist, darstellen muß, mit einer gegebenfalls im Reaktionsgemisch hergestellten Verbindung der allgemeinen Formel

$$H_2N - O - U - R_7 \qquad ,(V)$$

in der
X eine Carbonyl- oder Sulfonylgruppe und
$R_7$ eine in o-Stellung disubstituierte Arylgruppe wie eine 2,4,6-Trimethylphenyl- oder 2,4,6-Triisopropylphenylgruppe darstellen, umgesetzt wird oder

e) zur Herstellung einer Verbindung der allgemeinen Formel I, in der $R_1$ eine am Stickstoffatom durch eine Alkanoyl-, Alkylsulfonyl- oder Hydroxycarbonyl-alkylencarbonylgruppe substituierte Alkylsulfoximinogruppe darstellt, eine Verbindung der allgemeinen Formel

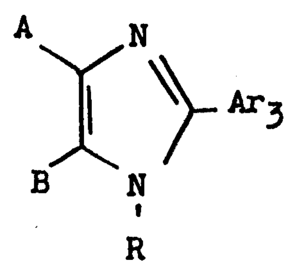

,(VI)

in der
A, B und R wie in den Ansprüchen 1 bis 5 definiert sind und $Ar_3$ die für Ar in den Ansprüchen 1 bis 5 erwähnten Bedeu-

tungen besitzt, wobei jedoch $R_1$ eine Alkylsulfoximinogruppe darstellen muß, mit einer Verbindung der allgemeinen Formel

$$R_8 \ - \ V \qquad ,(VII)$$

in der
$R_8$ eine Alkanoyl-, Alkylsulfonyl- oder Hydroxycarbonylalkylencarbonylgruppe und
V eine nukleofuge Austrittsgruppe wie ein Halogenatom oder eine Alkanoyloxygruppe darstellen, oder deren Ester und Anhydride, acyliert wird oder

f) zur Herstellung von Verbindungen der allgemeinen Formel I, in der $R_1$ eine Alkylsulfonyloxy-, Alkylsulfonylamino-, Alkoxycarbonylamino- oder N-Alkylaminocarbonyl-aminogruppe oder $R_4$ eine Alkanoylaminomethyl-, N-Ethylaminocarbonylamino-, N-n-Propylaminocarbonyl-amino-, N-Isopropylaminocarbonyl-amino- oder N-Alkyl-N-alkylaminocarbonyl-aminogruppe darstellt, eine Verbindung der allgemeinen Formel

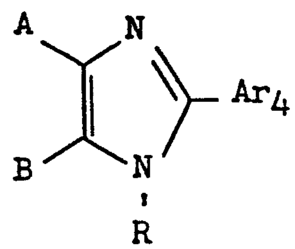

,(VIII)

in der
A, B und R wie in den Ansprüchen 1 bis 5 definiert sind und
$Ar_4$ die für Ar in den Ansprüchen 1 bis 5 erwähnten Bedeutungen besitzt, wobei jedoch $R_1$ eine Hydroxy- oder Aminogruppe oder $R_4$ eine Aminomethyl- oder Aminogruppe darstellen muß, mit einer Verbindung der allgemeinen Formel

$$R_9 \ - \ W \qquad ,(IX)$$

in der
$R_9$ eine Alkylgruppe mit 1 bis 3 Kohlenstoffatomen und W eine O=N=C- oder Halogensulfonylgruppe wie die Chlor- oder Bromsulfonylgruppe oder

$R_9$ eine Methyl- oder Ethylgruppe und W eine -CO-V-Gruppe darstellen, wobei V eine nucleofuge Austrittsgruppe wie ein Halogenatom oder eine Alkanoyloxygruppe darstellt, acyliert wird oder

g) zur Herstellung von Verbindungen der allgemeinen Formel I, in der R eine Alkylgruppe darstellt, eine Verbindung der allgemeinen Formel

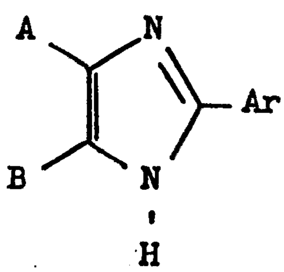
,(X)

in der
A, B und Ar wie in den Ansprüchen 1 bis 5 definiert sind, alkyliert wird und

gewünschtenfalls anschließend eine so erhaltene Verbindung der allgemeinen Formel I, in der $R_1$ eine Benzyloxygruppe darstellt, mittels Entbenzylierung in die entsprechende Hydroxyverbindung übergeführt wird, und/oder

eine so erhaltene Verbindung der allgemeinen Formel I, in der $R_4$ eine Cyangruppe darstellt, mittels Hydrolyse in die entsprechende Aminocarbonylverbindung übergeführt wird und/oder

eine erhaltene Verbindung der allgemeinen Formel I in ihr Säureadditionssalz, insbesondere in ihr physiologisch verträgliches Säureadditionssalz mit einer anorganischen oder organischen Säure, übergeführt wird.